(19) Europäisches Patentamt
European Patent Office
Office européen des brevets

(11) EP 1 421 935 A1

(12) EUROPEAN PATENT APPLICATION

(43) Date of publication:
26.05.2004 Bulletin 2004/22

(21) Application number: 02425687.7

(22) Date of filing: 08.11.2002

(51) Int Cl.[7]: **A61K 31/17**, A61K 31/18, A61K 31/03, A61K 31/415, A61K 31/401, A61K 31/405, A61K 31/192, A61K 31/44, A61K 38/05, A61K 31/197, A61K 31/198, A61K 38/07, A61K 38/06, A61K 31/4172, A61P 27/06

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicants:
- **Casini, Angela**
  **50019 Sesto Fiorentino (FI) (IT)**
- **Scozzafava, Andrea**
  **50019 Sesto Fiorentino (FI) (IT)**
- **Mincione, Francesco**
  **50134 Firenze (FI) (IT)**
- **Menabuoni, Luca**
  **50123 Firenze (FI) (IT)**
- **Supurant, Claudiu T.**
  **50019 Sesto Fiorentino (FI) (IT)**

(72) Inventors:
- **Casini, Angela**
  **50019 Sesto Fiorentino (FI) (IT)**
- **Scozzafava, Andrea**
  **50019 Sesto Fiorentino (FI) (IT)**
- **Mincione, Francesco**
  **50134 Firenze (FI) (IT)**
- **Menabuoni, Luca**
  **50123 Firenze (FI) (IT)**
- **Supurant, Claudiu T.**
  **50019 Sesto Fiorentino (FI) (IT)**

(74) Representative: **Barchielli, Giovanna et al**
**Ufficio Internazionale Brevetti**
**Ing. C. Gregorj S.p.A.**
**Via Dogana 1**
**20123 Milano (IT)**

(54) **Thiourea carbonic anhydrase inhibitors and their use in treatment of glaucoma**

(57) Carbonic anhydrase inhibitory compounds which incorporate a 4-sulfamoylphenylmethylthiourea scaffold are disclosed. The new compounds show strong affinities toward carbonic anhydrase isozymes I, II and IV and produce effective and prolonged reduction in intraocular pressure following topical ocular administration. The compounds are useful in treating glaucoma or ocular hypertension.

EP 1 421 935 A1

## Description

### BACKGROUND OF THE INVENTION

(1) Field of the Invention:

This invention relates to the use of carbonic anhydrase inhibitory compounds in treating eye diseases such as glaucoma, and in particular, to 4-sulfamoylphenylmethylthiourea compounds for topical use in reducing intraocular pressure and in treating glaucoma and ocular hypertension.

(2) Description of the Related Art:

Sulfonamide inhibitors of the zinc enzyme carbonic anhydrase (CA) are extensively used in clinical medicine and as diagnostic tools, their main applications being in the treatment of glaucoma, macular edema, epilepsy and other neurological disorders (Supuran et al., *Exp. Opin. Ther. Patents 12*:217-242, 2002; Supuran et al., *Curr. Med. Chem. Imm. Endoc. Metab. Agents 1*:61-97, 2001; Supuran et al., *Exp. Opin. Ther. Patents 10*:575-600, 2000). Several such drugs are presently available, such as the recently introduced topical sulfonamides dorzolamide **(1)** and brinzolamide **(2),** in addition to the classical, systemically acting inhibitors acetazolamide **(3)**, methazolamide **(4)**, ethoxzolamide **(5)** or dichlorophenamide **(6)**, which have been employed clinically for more than 45 years. The structures of these compounds are shown below.

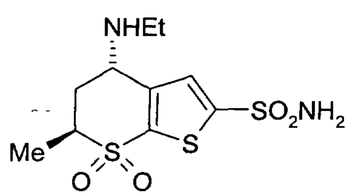

**1**

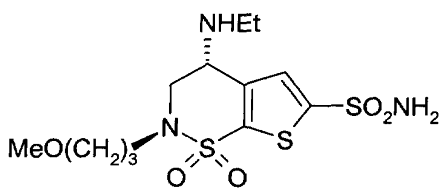

**2**

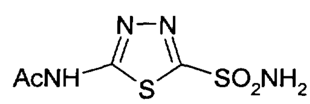

**3**

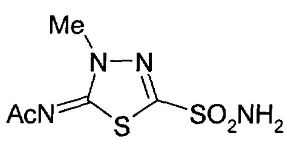

**4**

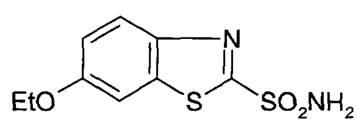

**5**

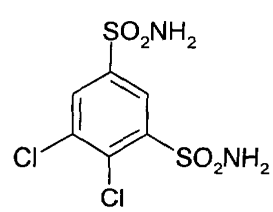

**6**

Systemic inhibitors possess many undesired side effects due to inhibition of several CA isozymes of which 14 are presently known to be present in higher vertebrates, in tissues other than the target one, i.e., the eye (more precisely, the ciliary processes of the eye). Furthermore, a number of other agents are presently used in physiological studies or as diagnostic tools rather than as antiglaucoma drugs (Supuran et al., 2002, *supra).* On the other hand, the two topically acting inhibitors, dorzolamide and brinzolamide, show a much more diminished number of side effects, together with an efficient reduction of intraocular pressure (IOP) due to inhibition of the enzymes (mainly isozymes CA II and CA IV) present in the ciliary processes, without appreciable inhibition of CA's from other tissues/organs (Supuran et al., 2002, *supra;* Supuran et al., 2001, *supra;* Supuran et al., 2000; Scozzafava et al., *J. Med. Chem* 42:2641-2650, 1999). Dorzolamide and brinzolamide can produce stinging sensations, burning or reddening of the eye, blurred vision, pruritus, and other local irritations, and, in the case of dorzolamide, this is likely to be due to the acidic pH of the dorzolamide solution. In order to reduce such inconveniences, numerous approaches have been reported to design topical antiglaucoma sulfonamides devoid of the above-mentioned side effects (see for example, Scozzafava et al., *J. Med. Chem. 42*:3690-3700, 1999; Scozzafava et al., *J. Enz. Inhib. 15*:533-546. (2000); Hies et al., *Bioorg. Med. Chem. 8:* 2145-2155, 2000).

Recently, Casini et al. reported on 4-sulfamoylphenylthiourea compounds which are prepared by reacting 4-isothiocyanatobenzenesulfonamide with amines, amino acids and oligonucleotides (Casini et al., *J. Med. Chem 43*: 4884-4892, 2000). The compounds were reported to have affinities toward isozymes I, II, and IC of carbonic anhydrase. The disclosure of this reference, however, was limited to specific 4-sulfamoylphenylthiourea compounds and there was no suggestion that any additional carbonic anhydrase inhibitory compounds could be prepared, other than those disclosed. Thus, there remains a continuing need for new carbonic anhydrase inhibitory compounds for use in reducing intraocular pressure and in treating glaucoma.

## BRIEF SUMMARY OF THE INVENTION

Accordingly, the inventors herein have succeeded in discovering a series of new sulfonamides which incorporate a 4-sulfamoylphenylmethylthiourea scaffold. These new thioureas show strong affinities towards isozymes I, II and IV of carbonic anhydrase. The new compounds reduce intraocular pressure when applied topically to the eye. As such, they are useful in the treatment and/or the prevention of conditions involving increased intraocular pressure, in particular glaucoma or ocular hypertension. The new compounds of the present invention can be used in the manufacture of a medicament for use in treating or preventing glaucoma or ocular hypertension.

Thus, in one embodiment, the present invention is directed to a thiourea compound or a salt thereof for use as a medicament for reduction of intraocular pressure and, in particular for treating glaucoma or ocular hypertension. The thiourea compound is one of a series of compounds which have the structure:

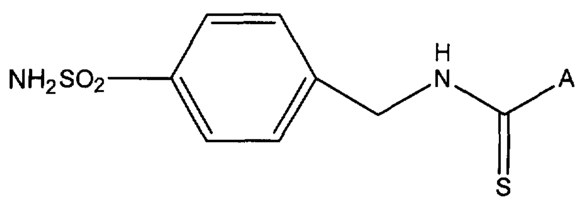

where A is selected from the group consisting of:

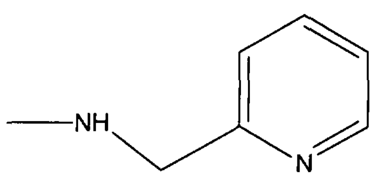
,

7

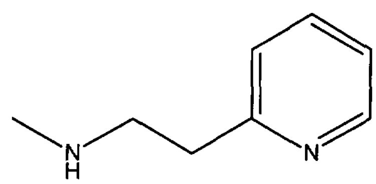
,

8

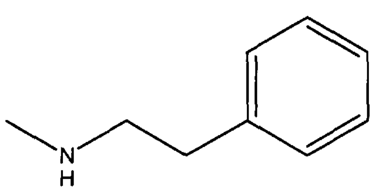
,

9

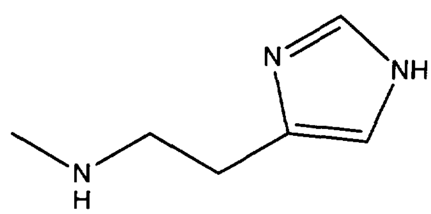
,

10

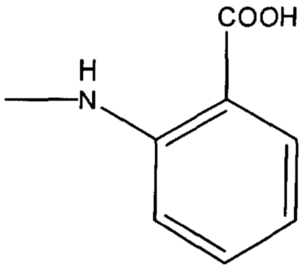

,

11

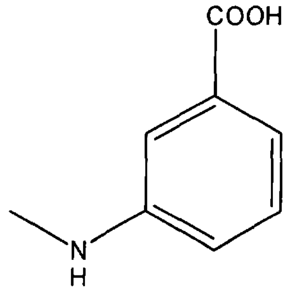

,

12

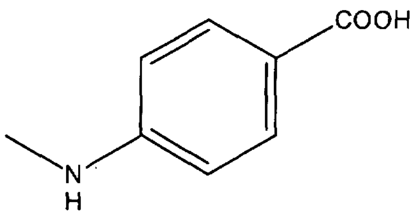

,

13

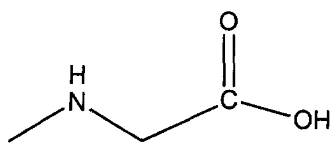

,

14

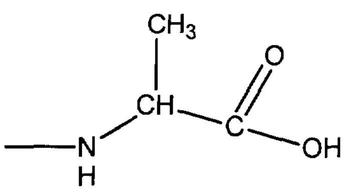

,

15

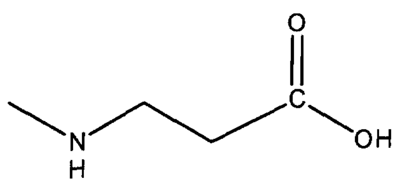

,

16

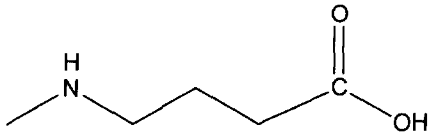

,

17

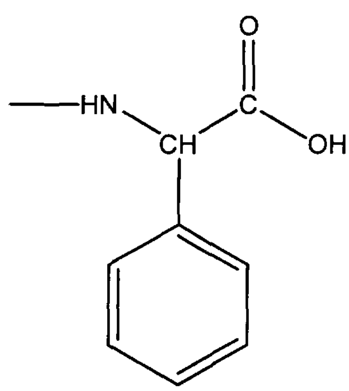

,

18

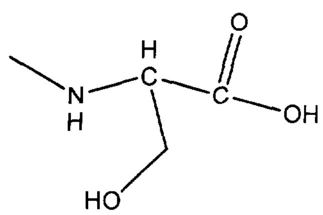
,

**19**

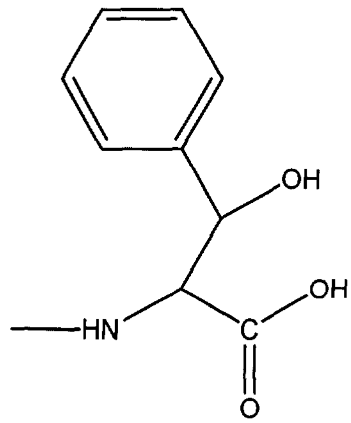
,

**20**

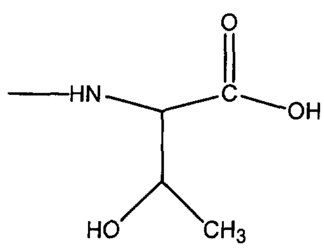
,

**21**

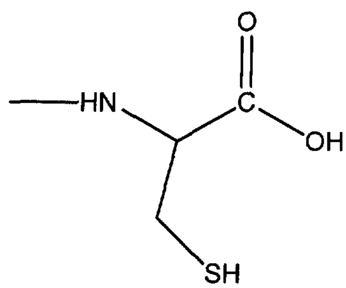
,

**22**

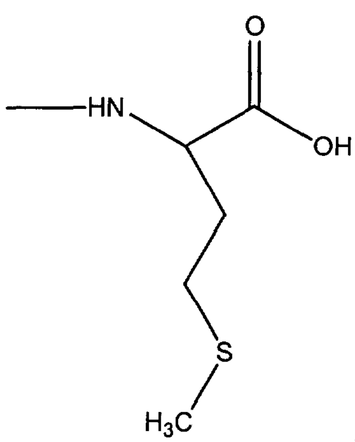
,

**23**

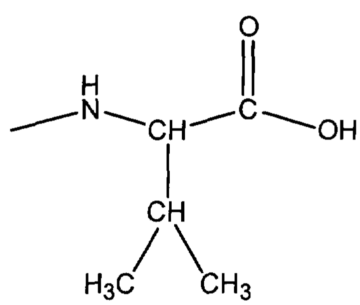
,

**24**

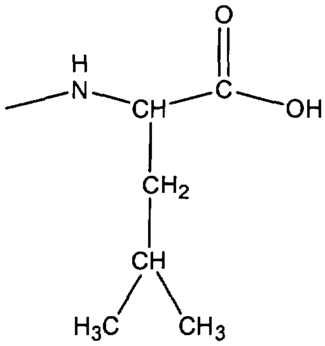
,

25

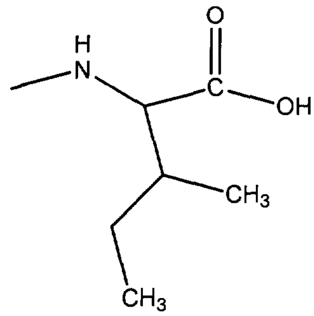
,

26

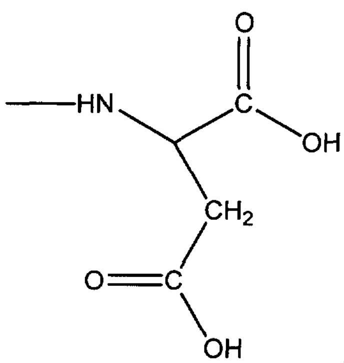
,

27

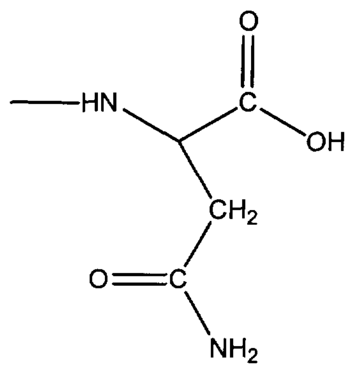
,

28

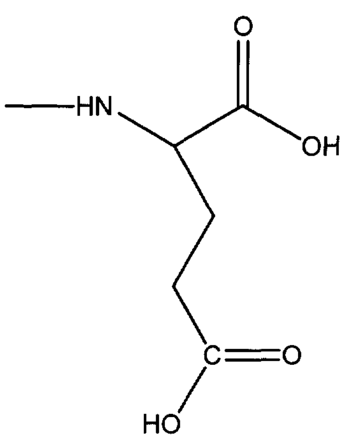

29

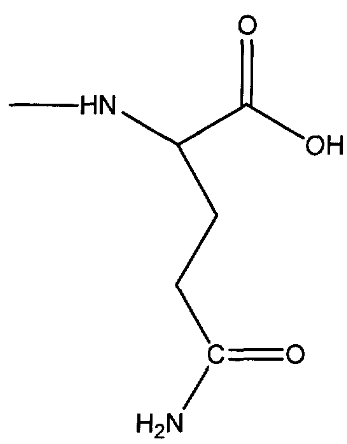

30

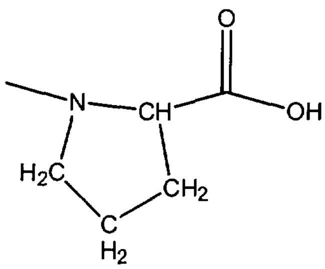

,

31

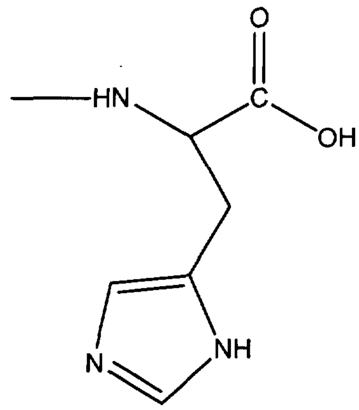

,

32

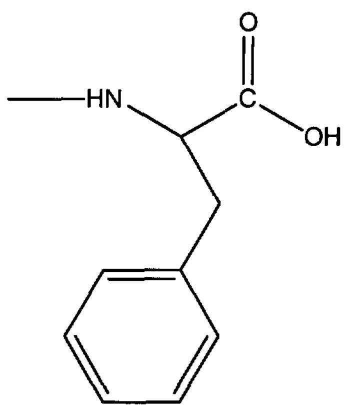

,

33

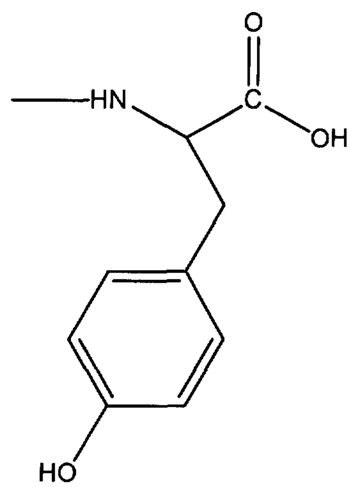

,

34

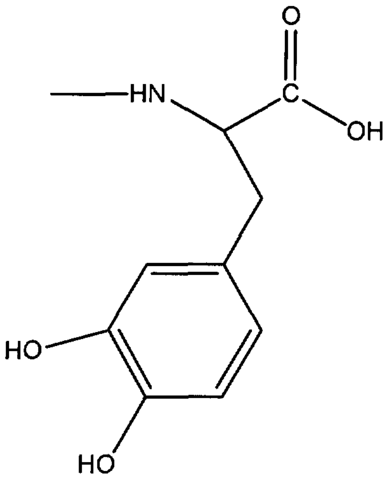
,

**35**

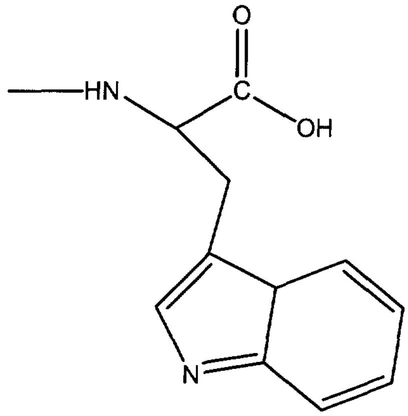
,

**36**

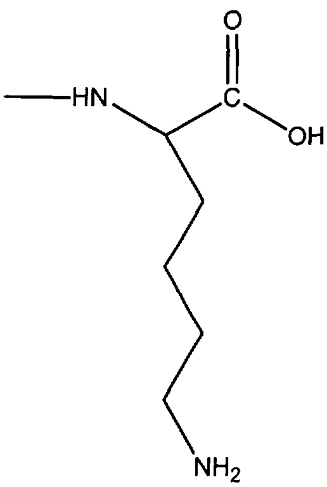
,

**37**

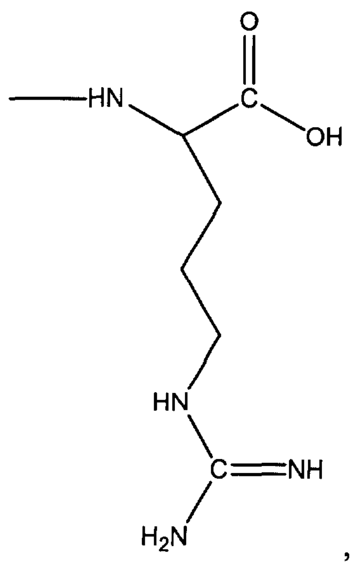
,

**38**

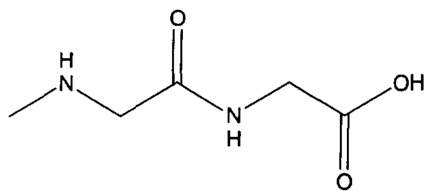
,

39

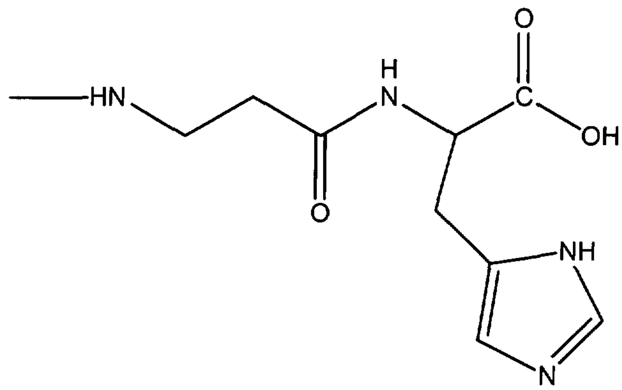
,

40

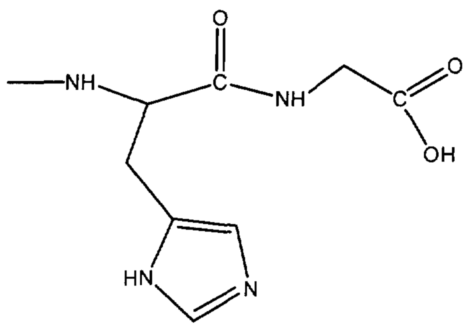
,

41

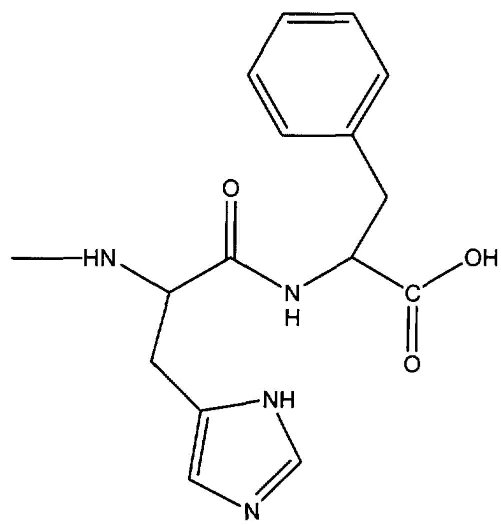
,

42

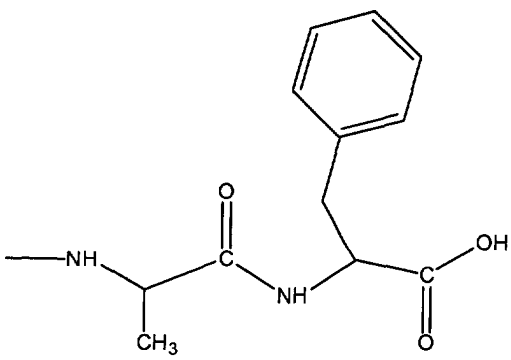

,

**43**

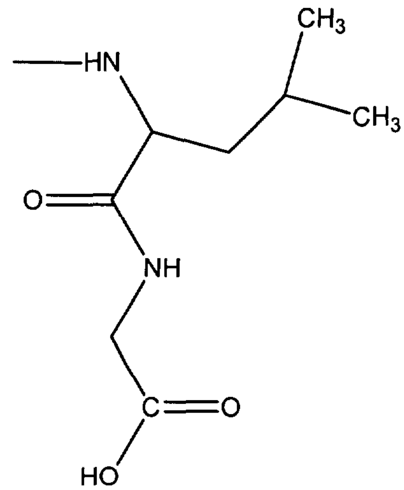

,

**44**

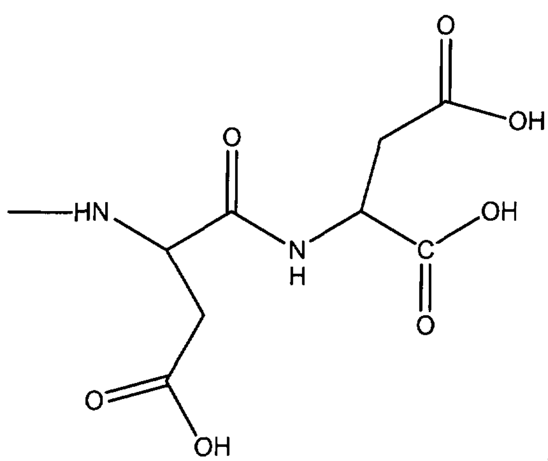

,

**45**

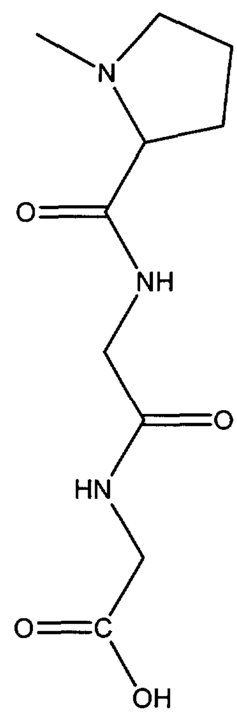

,

**46**

and

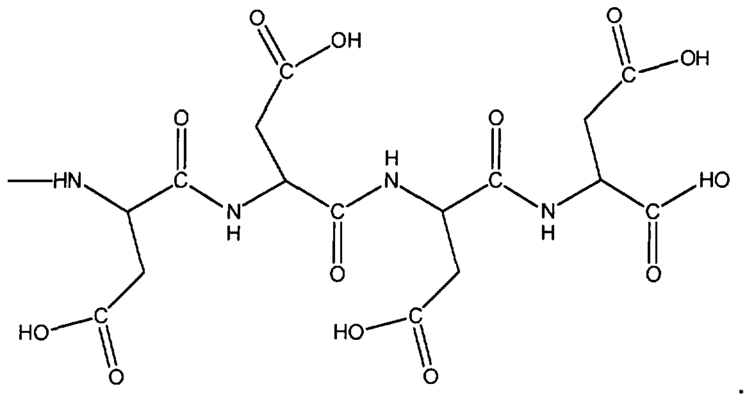

**47**

In one aspect, the thiourea compounds or salt thereof are for us in the manufacture of a medicament for the reduction in intraocular pressure.

In another aspect, the present invention is directed to an eye drop composition comprising a carbonic anhydrase inhibitory compound selected from the thiourea compounds above or a salt thereof. The carbonic anhydrase inhibitory compound is in an aqueous formulation suitable for topical application for reduction of intraocular pressure, and in particular, for treating glaucoma or ocular hypertension.

In another aspect of the present invention, the thiourea, carbonic anhydrase inhibitory compounds can be in an aqueous solution having a pH of from about 5.5 to about 7.4. In instances in which the compound is in the form of a salt, the salt can be a hydrochloride salt or a sodium carboxylate salt. The compound can be present in the aqueous solution at a concentration of about 2% weight per total weight of solution.

## BRIEF DESCRIPTION OF THE DRAWINGS

Figure 1 illustrates the CA II active site with the histidine cluster which comprises residues 64, 3 (not seen in the figure), 4, 17, 15 and 10; the zinc ion (central pink sphere); and the three histidine ligands (His 94, 96 and 119).

Figure 2 illustrates the effect of topically administered sulfonamide inhibitors (2 % water solutions) on the IOP of normotensive albino rabbits in which curve 1 represents dorzolamide (compound 1)(hydrochloride salt, pH 5.5); curve 2 represents compound B 13 (sodium salt, pH 7.0); curve 3 represents compound B 19 (sodium salt, pH 7.0); ); and curve 4 represents compound B20 (sodium salt, pH 7.0).

## DETAILED DESCRIPTION OF INVENTION

The present invention is directed to 4-sulfamoylphenylmethylthiourea compounds having intraocular pressure lowering properties. The compounds are useful in treating ocular hypertension, with potentially, little or no side effects due to acidic pH of the eye drop solution.

The synthesis of 4-isothiocyanato-benzenesulfonamide was reported by McKee and Bost, in the search for more effective antibacterial sulfonamides (McKee, and Bost *J. Am. Chem. Soc* 68:2506-2507, 1946). An approach similar to that of McKee and Bost can be used to synthesize isothiocyantomethylbenzenesulfonamide which by reaction with amines, amino acids and oligopeptides lead to a series of new sulfonamides incorporating a 4-sulfamoyl-methylthiourea scaffolding. Accordingly isothiocyanate **B** as well as the corresponding thioureas **B7-B47** can be prepared. The reaction scheme which could be used for the synthesis of 4-isothiocyanatomethyl-benzenesulfonamide **B** and of the thioureas **B7-B47** is shown below.

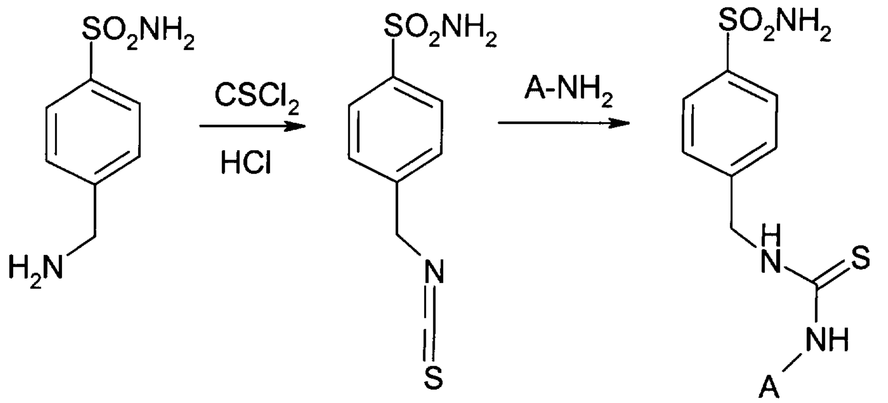


B

B7-B47

**Scheme 1.**

Amines **87-847** are shown below.

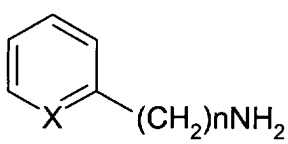

**7:** X=N; n = 1

**8:** X=N; n = 2

**9:** X=CH; n =2

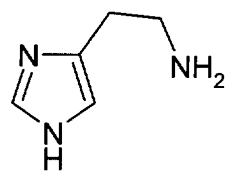

**10**

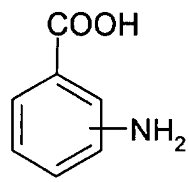

**11:** $2\text{-}NH_2$

**12:** $3\text{-}NH_2$

**13:** $4\text{-}NH_2$

**14:** Gly; **15:** Ala; **16:** beta-Ala; **17:** GABA; **18:** alpha-Ph-Gly; **19:** Ser; **20:** beta-Ph-Ser **21:** Thr; **22:** Cys; **23:** Met; **24:** Val; **25:** Leu; **26:** Ile; **27:** Asp; **28:** Asn; **29:** Glu; 30: Gln **31:** Pro; **32:** His; **33:** Phe; **34:** Tyr; **35:** DOPA; **36:** Trp; **37:** Lys; **38:** Arg; **39:** GlyGly **40:** beta-AlaHis; **41:** HisGly; **42:** HisPhe; **43:** AlaPhe; **44:** LeuGly; **45:** AspAsp **46:** ProGlyGly; **47:** $(Asp)_4$

The newly obtained derivatives are numbered herein as **B7** to **B47,** denoting that the $4\text{-}H_2NO_2S\text{-}C_6H_4\text{-}CH_2NHCSNH\text{-}$ group is attached to the corresponding moiety of the starting nucleophile **7-47.** For instance, **B9** is the thiourea obtained by the reaction of phenethylamine **9** with 4-isothiocyanatomethyl-benzenesulfonamide **B; B14** is the thiourea obtained by reaction of glycine **14** with 4-isothiocyanatomethyl-benzenesulfonamide **B; B40** is the thiourea obtained by reaction of carnosine **40** with 4- isothiocyanatomethyl-benzenesulfonamide **B;** etc. Structures **B9; B14;** and **B40** are shown below.

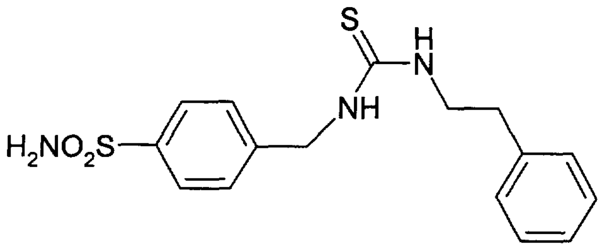

**B9**

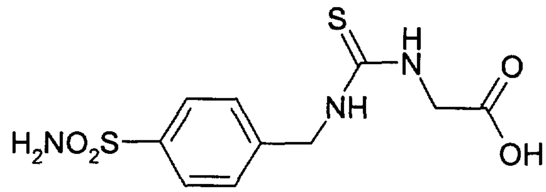

**B14**

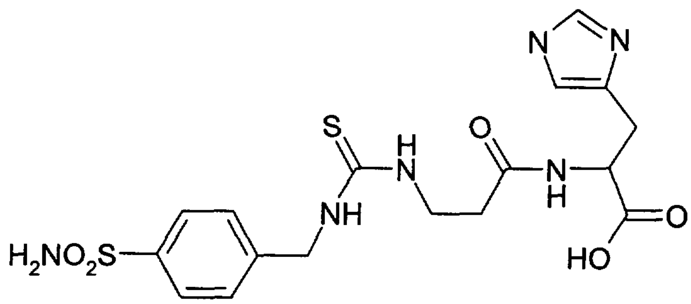

**B40**

Thus, compound **B** could be synthesized from homosulfanilamide hydrochloride and thiophosgene according to Scheme 1 and the subsequent reaction with a large number of amines, amino acids and oligopeptides, of type **7-47,** would produce the new thioureas **B7-B47.**

The nucleophiles for use in the preparation of the new compounds **(7-47)** are chosen in such a manner as to contain water-solubilizing moieties in the presence of acids/bases, such as the pyridine or imidazole moieties in the case of **7, 8** and **10** (hydrochlorides [Scozzafava et al, *J. Enz. Inhib.,15:533-546,* 2000; Ilies et al., *Bioorg. Med. Chem.* 8:2145-2155, 2000] or triflates *[Id.]* of **B7**, **B8** or **B10** would presumably lead to water soluble CA inhibitors, but the compound unable to form such a salt - **B9** - would be expected to confirm the hypothesis mentioned above). Thioureas obtained from other nucleophiles (**B11** - **B47**) on the other hand easily form water soluble sodium salts due to the presence of at least one carboxyl group in their molecules. It was previously noted in this laboratory that sulfonamides incorporating carboxylate moieties in their molecules might interact with a histidine cluster at the entrance of the hCA II active site (Supuran et al., *Curr. Med. Chem. Imm., Endoc. Metab. Agents,* 1:61-97, 2001; Briganti et al., *Biochemistry* 36:10384-10392, 1997). This is the isozyme that plays the most important function in aqueous humor secretion (Supuran et al., (2002) *Exp. Opin. Ther. Patents12:217-242,* 2002; Supuran et al., *Curr. Med. Chem. - Imm., Endoc. Metab. Agents, 1*:61-97,2001; Supuran et al., *Exp. Opin. Ther. Patents 10*:575-600, 2000). This cluster as shown in Figure 1, comprises the residues His 64 (at the center of the active site cavity), His 4 and His 3 (at the rim) as well as several residues on the external surface of the enzyme, near the entrance to the active site cavity (His 10, His 15 and His 17; hCA I numbering) (Briganti et al., *Biochemistry 36*:10384-10392, 1997).

All imidazolic side chains of these residues may interact, particularly when in charged state, as imidazolium ions, with negatively charged inhibitors. Such inhibitors can contain, for instance, carboxylate anions, leading in this way to a supplementary stabilization of the enzyme-inhibitor adduct (Casini et al., *J. Med. Chem 43*:4884-4892, 2000). For this reason, compounds of this type are designed and prepared in this study.

The greatest majority of these new compounds contain one $COO^-$ moiety which forms a sodium salt solution, but derivatives with two **(B45)** or four **(B47)** carboxylate moieties are also included. Many such derivatives show very good CA inhibitory properties as discussed below.

<u>EXAMPLE 1</u>

This example illustrates a method for the preparation of **4**-isothiocyanatomethyl-benzenesulfonamide (compound **B).**

The method of McKee and Bost (McKee and Bost, 1946, *supra)* is modified as follows. An amount of 22.2 g (0.1 mol) of homosulfanilamide hydrochloride is dissolved in 200 mL of water and 10 mL of concentrated hydrochloric acid solution is added, together with 11.2 g (0.1 mol) of thiophosgene. Stirring is begun immediately until all the red color of thiophosgene disappears (around 3.5-4 h) and a white crystalline precipitate forms. The product is filtered and recrystallized from acetone-water (1:1, v/v). The yield is 95 % (m.p. 179-81°C).

EXAMPLE 2

This example illustrates a general procedure for the preparation of compounds B7-B47.

An amount of 0.53g (2.5 mMoles) of 4-isothiocyanatomethyl-benzenesulfonamide **A** and the stoichiometric amount of nucleophile **7-47** is suspended in 50-100 mL of dry acetone or acetonitrile and heated at reflux for 2-8 hours (TLC control). The solvent is evaporated, and the crude product either recrystallized from ethanol or ethanol-water (for the largest majority of thioureas described here), or purified by preparative HPLC in the case when the reaction mixture contained appreciable amounts of impurities (as evidenced by TLC). This is particularly the case for the oligopeptidyl thioureas B39-B47. Conditions used for the purification are: $C_{18}$ reversed-phase μ-Bondapack or Dynamax-60A (25x250 mm) preparative columns; 90 % acetonitrile/ 8% methanol/ 2 % water, 30 mL/min.

EXAMPLE 3

This example illustrates the properties of 1-(4-Sulfamoylphenylmethyl)-3-[(1H-imidazol)-4-yl-ethyl]-thiourea (histamine derivative) **B10** which are as follows: white crystals, m.p. 173-4 °C (acetone-water 1:1, v/v). IR (KBr), $cm^{-1}$: 1150 ($SO_2^{sym}$), 1245 (thioamide III), 1375 ($SO_2^{as}$), 1550 (thioamide II), 1680 (thioamide I), 3060 (NH); $^1$H-NMR (DMSO-$d_6$), δ, ppm: 2.49 (t, 2H, J = 7.0, Hst $CH_2$); 2.99 (t, 2H, J = 7.0, Hst CSNHC*$H_2$*); 4.49 (d, 2H, $^3J_{HH}$=6.0, $H_2NO_2SC_6H_4$-C*$H_2$*); 7.34 (m, 1H, imidazole CH); 7.52 (s, 2H, $SO_2NH_2$), 7.65 (d, $^3J_{HH}$=8.1, 2H, $\underline{H}_{ortho}$ of $H_2NO_2SC_6H_4$), 7.94 (d, $^3J_{HH}$=8.1, 2H, $\underline{H}_{meta}$ of $H_2NO_2SC_6H_4$); 8.21 (br s, 2H, NHCSNH); 8.35 (s, 1H, imidazole CH); 8.80 (s, 1H, imidazole NH); $^{13}$C-NMR (DMSO-$d_6$), δ, ppm: 33.23 (s, $\underline{C}H_2$ of Hst); 37.64 (s, $\underline{C}H_2$ of Hst); 41.58 ($CH_2$); 123.70 (s, C-4 of Hst); 130.93 (s, $\underline{C}_{meta}$ of $H_2NO_2SC_6H_4$), 134.32 (s, C-5 of Hst); 135.13 (s, $\underline{C}_{ortho}$ of $H_2NO_2SC_6H_4$), 137.30 (s, C-2 of Hst); 139.43 (s, NH$\underline{C}$SNH), 144.68 (s, $\underline{C}_{ipso}$ of $H_2NO_2SC_6H_4$), 148.14 (s, $\underline{C}_{para}$ of $H_2NO_2SC_6H_4$); Anal.: C, 45.94; H, 5.32; N, 20.36 %; $C_{13}H_{17}NsO_2S_2$ requires C, 46.00; H, 5.05; N, 20.63 %.

EXAMPLE 4

This example illustrates the properties of 1-(4-Sulfamoylphenylmethyl)-3-(carboxymethyl)-thiourea (glycine derivative) **B14** which are as follows: white crystals, m.p. 168-9 °C (acetone-water 1:2, v/v). IR (KBr), $cm^{-1}$: 1153 ($SO_2^{sym}$), 1240 (thioamide III), 1375 ($SO_2^{as}$), 1550 (thioamide II), 1682 (thioamide I), 1754 (COOH), 3065 (NH); $^1$H-NMR (DMSO-$d_6$), δ, ppm: 3.67 (s, 2H, C$\underline{H_2}$ of Gly); 4.47 (d, 2H, $^3J_{HH}$=6.0, $H_2NO_2SC_6H_4$-C*$H_2$*); 7.54 (s, 2H, $SO_2NH_2$), 7.63 (d, $^3J_{HH}$=8.1, 2H, $\underline{H}_{ortho}$ of $H_2NO_2SC_6H_4$), 7.94 (d, $^3J_{HH}$=8.1, 2H, $\underline{H}_{meta}$ of $H_2NO_2SC_6H_4$); 8.21 (br s, 2H, NHCSNH); 10.63 (br s, 1H, COOH); $^{13}$C-NMR (DMSO-$d_6$), δ, ppm: 40.81 (s, $\underline{C}H_2$ of Gly); 41.62 ($CH_2$); 131.53 (s, $\underline{C}_{meta}$ of $H_2NO_2SC_6H_4$), 135.49 (s, $\underline{C}_{ortho}$ of $H_2NO_2SC_6H_4$), 139.76 (s, NH$\underline{C}$SNH), 144.24 (s, $\underline{C}_{ipso}$ of $H_2NO_2SC_6H_4$), 147.57 (s, $\underline{C}_{para}$ of $H_2NO_2SC_6H_4$); 179.83 (COOH); Anal.: C, 39.30; H, 4.68; N, 13.57 %; $C_{10}H_{13}N_3O_4S_2$ requires C, 39.55; H, 4.32; N, 13.85%.

EXAMPLE 5

This example illustrates the properties of 1-(4-Sulfamoylphenylmethyl)-3-(1-carboxy-2-methyl-butyl)-thiourea (isoleucine derivative) **B26** which are as follows: white crystals, m.p. 170-2 °C (acetone-water 1:1, v/v). IR (KBr), $cm^{-1}$: 1151 ($SO_2^{sym}$), 1244 (thioamide III), 1376 ($SO_2^{as}$), 1557 (thioamide II), 1676 (thioamide I), 1750 (COOH), 3065 (NH); $^1$H-NMR (DMSO-$d_6$), δ, ppm: 1.15 (d, $^3J_{HH}$=6.5, 3H, C$\underline{H}_3$ of Ile), 1.21 (t, 3H, $^3J_{HH}$=6.7, $CH_3$ of Et moiety of Ile); 1.54 (m, 2H, $CH_2$ of Ile); 3.22 (m, 1H, EtC$\underline{H}$(Me)- of Ile); 3.75 (m, 1H, NHC$\underline{H}$CO of Ile), 4.48 (d, 2H, $^3J_{HH}$=6.0, $H_2NO_2SC_6H_4$-C*$H_2$*); 7.51 (s, 2H, $SO_2NH_2$), 7.66 (d, $^3j_{HH}$=8.0, 2H, $\underline{H}_{ortho}$ of $H_2NO_2\underline{S}C_6H_4$), 7.92 (d, $^3J_{HH}$=8.0, 2H, $\underline{H}_{meta}$ of $H_2NO_2SC_6H_4$); 8.20 (br s, 2H, NHCSNH); 10.42 (br s, 1H, COOH); $^{13}$C-NMR (DMSO-$d_6$), δ, ppm: 21.78 (s, CH$\underline{C}H_3$ of Ile), 22.57 (s, $\underline{C}H_3CH_2$ of Ile); 31.42 (s, $\underline{C}H_2$ of Ile); 34.58 (s, $\underline{C}H(CH_3)_2$ of Leu), 41.57 ($CH_2$); 46.36 (s, Et$\underline{C}$H(Me)- of Ile), 55.12 (s, NH$\underline{C}HCH_2$ of Ile), 131.43 (s, $\underline{C}_{meta}$ of $H_2NO_2SC_6H_4$), 135.58 (s, $\underline{C}_{ortho}$ of $H_2NO_2SC_6H_4$), 139.75 (s, NH$\underline{C}$SNH), 144.28 (s, $\underline{C}_{ipso}$ of $H_2NO_2SC_6$H4), 147.64 (s, $\underline{C}_{para}$ of $H_2NO_2SC_6H_4$); 179.16 (COOH); Anal.: C, 46.90; H, 5.75; N, 11.50 %; $C_{14}H_2N_3O_4S_2$ requires C, 46.78; H, 5.89; N, 11.69 %.

EXAMPLE 6

This example illustrates the properties of 1-(4-Sulfamoylphenylmethyl)-3-(1-carboxy-2-phenyl-ethyl)-thiourea (phenylalanine derivative) **B33** which are as follows: white crystals, m.p. 208-9 °C (ethanol-water 1:1, v/v). IR (KBr), $cm^{-1}$: 1148 ($SO_2^{sym}$), 1243 (thioamide III), 1376 ($SO_2^{as}$), 1550 (thioamide II), 1684 (thioamide I), 1751 (COOH), 3065 (NH); $^1$H-NMR (DMSO-$d_6$), δ, ppm: 3.10-3.55 (m, 2H, $CH_2CH$ of Phe), 4.08 (dd, $^3J_{HH}$=5.0, $^3J_{HH}$=7.8, 1H, $CH_2CH$ of Phe), 4.49 (d, 2H, $^3J_{HH}$=6.0, $H_2NO_2SC_6H_4$-$CH_2$); 7.29-7.51 (m, 5H, $H_{arom}$ ofPhe);7.57 (s, 2H, $SO_2NH_2$), 7.63 (d, $^3J_{HH}$=8.1, 2H, $H_{ortho}$ of $H_2NO_2SC_6H_4$), 7.96 (d, $^3J_{HH}$=8.1, 2H, $H_{meta}$ of $H_2NO_2SC_6H_4$); 8.20 (br s, 2H, NHCSNH); 10.71 (br s, 1H, COOH); $^{13}$C-NMR (DMSO-$d_6$), δ, ppm: 41.23 (s, $CH_2CH$ of Phe), 41.64 ($CH_2$); 59.30 (s, $CH_2CH$ of Phe), 131.56 (s, $C_{meta}$ of $H_2NO_2SC_6H_4$), 133.70 (s, $C_{meta}$ of Phe), 134.24 (s, $C_{ortho}$ of Phe), 135.50 (s, $C_{ortho}$ of $H_2NO_2SC_6H_4$), 139.61 (s, NHCSNH), 141.58 (s, $C_{ipso}$ of Phe), 144.23 (s, $C_{ipso}$ of $H_2NO_2SC_6H_4$), 145.45 (s, $C_{para}$ of Phe), 147.37 (s, $C_{para}$ of $H_2NO_2SC_6H_4$); 179.25 (COOH); Anal.,: C, 51.70; H, 4.67; N, 10.54 %; $C_{17}H_{19}N_3O_4S_2$ requires C, 51.89; H, 4.87; N, 10.68 %.

EXAMPLE 7

This example illustrates the properties of 4-Sulfamoylphenylmethylthioureido-β-alanyl-histidine (carnosine derivative), **B40** which are as follows: white crystals, m.p. 155-7 °C. IR (KBr), $cm^{-1}$: 1156 ($SO_2^{sym}$), 1240 (thioamide III), 1287 (amide I), 1375 ($SO_2^{as}$), 1540 (amide I), 1550 (thioamide II), 1683 (thioamide I), 1720 (amide I), 1750 (COOH), 3065 (NH); $^1$H-NMR (DMSO-$d_6$), δ, ppm: 2.79-2.88 (m, 2H, $CH_2$ of □-Ala), 3.11-3.26 (m, 2H, $CH_2$ of □-Ala), 3.34-3.45 (m, 2H, $CHCH_2$ of His), 4.45 (d, 2H, $^3J_{HH}$=6.0, $H_2NO_2SC_6H_4$-$CH_2$); 4.57-4.63 (m, 1H, $CHCH_2$ of His), 7.33 (s, 1H, CH-5 of His), 7.54 (s, 2H, $SO_2NH_2$), 7.63 (d, $^3J_{HH}$=8.1, 2H, Hortho of $H_2NO_2SC_6H_4$), 7.94 (d, $^3J_{HH}$=8.1, 2H, $H_{meta}$ of $H_2NO_2SC_6H_4$); 8.21 (br s, 2H, NHCSNH); 8.29 (br s, 1H, CONH); 8.35 (s, 1H, CH-2 of His); 8.80 (s, 1H, imidazole NH from His)10.63 (br s, 1H, COOH); $^{13}$C-NMR (DMSO-$d_6$), δ, ppm: 33.27 (s, $CH_2$ of His), 37.50 (s, $NHCH_2CH_2$ of □-Ala), 40.69 (s, $CH_2CH_2CO$ of □-Ala), 41.55 ($CH_2$); 59.48 (s, $CHCH_2$ of His), 122.21 (s, C-4 of His), 131.56 (s, $C_{meta}$ of $H_2NO_2SC_6H_4$), 134.28 (s, C-5 of His), 135.09 (s, $C_{ortho}$ of $H_2NO_2SC_6H_4$), 137.57 (s, C-2 ofHis), 139.33 (s, NHCSNH), 144.16 (s, $C_{ipso}$ of $H_2NO_2SC_6H_4$), 147.24 (s, $C_{para}$ of $H_2NO_2SC_6H_4$); 175.60 (s, $CH_2CO$ of □-Ala), 180.09 (COOH); Anal.: C, 45.07; H, 4.62; N, 18.30 %; $C_{17}H_{22}N_6O_5S_2$ requires C, 44.92; H, 4.88; N, 18.49%.

EXAMPLE 8

This example illustrates physico-chemical properties for selected compounds.

The determination of solubility in aqueous buffer is performed as follows. A standard solution is prepared by dissolving a precisely weighted amount (generally 1 mg) of inhibitor in 10 mL of methanol. The UV absorption maximum of each compound is determined (with a Cary 3 spectrophotometer) eventually diluting the solution (with MeOH) as necessary. A saturated solution of each compound is then prepared by stirring magnetically a small volume of 0.039 M phosphate buffer (pH 7.4) in the presence of excess inhibitor for 3 hours. The obtained saturated solution is filtered in order to remove solid compound through a Millipore 0.45 μm filter and scanned by UV at the wavelength of the absorption maximum previously determined. Total solubility is determined by the relationship: C' = A'C/A, where C = concentration of standard solution (mg/mL); A = absorbance of standard solution; A' = absorbance of the saturated solution; C' = concentration of the saturated solution (mg/mL). (Supuran et al., *Eur. J. Med. Chem. 33*:73 9-751, 1998)

The determination of partition coefficient is performed as follows. Chloroform - buffer partition coefficients are obtained by equilibrating the test compound between chloroform and 0.1-ionic strength pH 7.4 phosphate buffer. The concentration in each phase is determined by UV spectrophotometry or HPLC. (Supuran et al., *Eur. J. Med. Chem.* 33:739-751, 1998).

The determination of transcorneal penetration of drugs is performed as follows. The method of Maren et al. (Maren et al., *Mol. Pharmacol 44*:941-906, 1993) with the modifications of Pierce's group (Pierce et al., *Proc. Soc. Exp. Biol. Med.203:360-365,* 1993; Sharir et al., *Exp. Eye Res. 58*:107-116, 1994) (for the HPLC assay of sulfonamides) are used. Excised rabbit corneas with either intact or denuded epithelium are used in these experiments. The pH is 7.4 and exposed area is 1.2 $cm^2$. Concentrations of drug of 40 - 2000 μM are placed in the epithelial chamber and samples of fluid are collected from the endothelial chamber at different intervals, up to 4 hr. Both chambers contained 6 mL. Drugs present in these fluids are assayed both by the HPLC method of Pierce et al., (Pierce et al., 1993, *supra;* Sharir et al., 1994, *supra)* or enzymatically (Supuran et al., *Eur. J. Med. Chem. 33:73 9-751,* 1998.). The results of the drug analyses are used to calculate the rate constant of transfer across the cornea ($k_{in}$). As described by Pierce (Pierce et al., 1993, *supra;* Sharir et al., 1994, *supra),* this value is determined by using the formula:

$$k_{in}.\ (x\ 10^3\ hr^{-1}) = [drug]_{endo}/[drug]_{epi}\ x\ 60/t\ x\ 1000$$

where $[drug]_{endo}$= concentration of drug on endothelial side; $[drug]_{epi}$ = concentration of drug on epithelial side; t = time (in min).

The Solubility, chloroform-buffer partition coefficients and in vitro corneal permeability of some classical and new sulfonamide CA inhibitors are shown in Table 1.

Table 1.

| Compound epithelium | Solubility[a] mM | Log P[b] | $k_{in} \times 10^3$ ($hr^{-1}$)[c] | |
|---|---|---|---|---|
| | | | Cornea intact | No |
| **Dorzolamide [d]** | 60 | 2.0[e] | 3.0 | 5.2 |
| **Acetazolamide** | 3.2 | 0.001 | 0.37 | 7.0 |
| **Methazolamide** | 12 | 0.06 | 1.90 | 13 |
| **Ethoxzolamide** | 0.04 | 1.40 | 27 | 40 |
| **B11[e]** | 41 | 1.238 | 2.9 | 8.3 |
| **B12[e]** | 40 | 1.313 | 3.0 | 8.7 |
| **B13[e]** | 46 | 1.428 | 3.3 | 9.1 |
| **B18[e]** | 59 | 3.721 | 5.6 | 12.0 |
| **B19[e]** | 70 | 1.662 | 3.3 | 8.5 |
| **B20[e]** | 51 | 2.518 | 4.9 | 10.7 |
| **B22[e]** | 65 | 0.124 | 0.4 | 0.9 |
| **B40[e]** | 57 | 0.933 | 2.8 | 5.9 |

[a]Solubility in pH 7.40 buffer, at 25°C.
[b]Chloroform-buffer partition coefficient.
[c]Determined as described in ref.[28,29]
[d]As hydrochloride, at pH 5.8.
[e]As sodium salts, at pH 7.0.

EXAMPLE 9

This example illustrates the carbonic anhydrase inhibitory properties of the compounds.

Human CA I and CA II cDNAs are expressed in *Escherichia coli* strain BL21 (DE3) from the plasmids pACA/hCA I and pACA/hCA II described by Lindskog's group (Lindskog et al., In *Carbonic anhydrase - From biochemistry and genetics to physiology and clinical medicine,* Botrè et al., Eds., VCH, Weinheim, pp. 1-13, 1991). Cell growth conditions are described by Behravan et al. (Behravan et al., *Eur. J. Biochem 190*:351-357, 1990). Enzymes are purified by affinity chromatography according to the method of Khalifah et al. (Khalifah et al., *Biochemistry 16:2241*-2247, 1977). Enzyme concentrations are determined spectrophotometrically at 280 nm, utilizing a molar absorptivity of 49 $mM^{-1}.cm^{-1}$ for CA I and 54 $mM^{-1}.cm^{-1}$ for CA II, respectively, based on $M_r$= 28.85 kDa for CA I, and 29.3 kDa for CA II, respectively.[13,14] bCA IV is isolated from bovine lung microsomes as described by Maren et al, and its concentration is been determined by titration with ethoxzolamide (Maren et al., *Mol. Pharmacol 44*:901-906, 1993). Initial rates of 4-nitrophenylacetate hydrolysis catalysed by different CA isozymes are monitored spectrophotometrically, at 400 nm, with a Cary 3 instrument interfaced with an IBM compatible PC.[16] Solutions of substrate are prepared in anhydrous acetonitrile; the substrate concentrations varied between $2.10^{-2}$ and $1.10^{-6}$ M, working at 25°C. A molar absorption coefficient $\varepsilon$ of 18,400 $M^{-1}.cm^{-1}$ is used for the 4-nitrophenolate formed by hydrolysis, in the conditions of the experiments (pH 7.40), as reported in the literature. [16] Non-enzymatic hydrolysis rates are always subtracted from the observed rates. Duplicate experiments are done for each inhibitor concentration, and the values reported herein are the mean of such results. Stock solutions of inhibitor (1 mM) are prepared in distilled-deionized water with 10-20% (v/v) DMSO (which is not inhibitory at these concentrations) and dilutions up to 0.01 nM are done thereafter with distilled-deionized water. Inhibitor and enzyme solutions are preincubated together for 10 min at room temperature prior to assay, in order to allow for the formation of the E-I complex. The inhibition constant $K_I$ is determined as described by Pocker and Stone (Pocker et al., *Biochemistry 6*:668-678, 1967). Enzyme concentrations are 3.6 nM for CA II, 9.1 nM for CA I and 30 nM for CA IV (this isozyme has a decreased esterase activity [Baird et al., *Biochemistry 36*:2669-2678, 1997] and higher concentrations have to be used for the measurements).

CA inhibition data against three isozymes, hCA I, hCA II and bCA IV (h = human; b = bovine isozyme) for the new compounds and standard inhibitors are shown in Table 2

Table 2

| No | Inhibitor | $K_I$ (nM) | | |
|---|---|---|---|---|
| | | hCA I[a] | hCA II[a] | bCA IV[b] |
| Dorzolamide **1** | | 50000 | 9 | 43 |
| Acetazolamide **3** | | 900 | 12 | 220 |
| Methazolamide **4** | | 780 | 14 | 240 |
| Ethoxzolamide **5** | | 25 | 8 | 13 |
| Dichlorophenamide **6** | | 1200 | 38 | 380 |
| | **A** | 5000 | 185 | 300 |
| | **B** | 4300 | 162 | 270 |
| | **B7** | 124 | 43 | 74 |
| | **B8** | 120 | 38 | 75 |
| | **B9** | 126 | 39 | 76 |
| | **B10** | 88 | 30 | 52 |
| | **B11** | 54 | 11 | 23 |
| | **B12** | 38 | 10 | 29 |
| | **813** | 47 | 9 | 21 |
| | **B14** | 56 | 17 | 34 |
| | **B15** | 48 | 10 | 29 |
| | **B16** | 39 | 8 | 28 |
| | **B17** | 40 | 10 | 27 |
| | **B18** | 40 | 5 | 12 |
| | **B19** | 23 | 2 | 9 |
| | **B20** | 20 | 1 | 7 |
| | **B21** | 24 | 2 | 13 |
| | **B22** | 21 | 3 | 8 |
| | **B23** | 19 | 3 | 10 |
| | **B24** | 20 | 4 | 9 |
| | **B25** | 17 | 3 | 11 |
| | **B26** | 18 | 4 | 8 |
| | **B27** | 27 | 7 | 15 |
| | **B28** | 22 | 5 | 9 |
| | **B29** | 39 | 10 | 23 |
| | **B30** | 36 | 9 | 21 |
| | **B31** | 89 | 21 | 55 |
| | **B32** | 33 | 6 | 14 |
| | **B33** | 27 | 5 | 14 |
| | **B34** | 24 | 3 | 9 |
| | **B35** | 30 | 6 | 14 |
| | **B36** | 42 | 9 | 17 |
| | **B37** | 73 | 10 | 34 |
| | **B38** | 103 | 18 | 56 |
| | **B39** | 50 | 13 | 26 |
| | **B40** | 22 | 4 | 9 |
| | **B41** | 12 | 1 | 5 |
| | **B42** | 19 | 2 | 7 |
| | **B43** | 21 | 3 | 10 |

[a]Human (cloned) isozymes;
[b]From bovine lung microsomes, by the esterase method.

Table 2 (continued)

| No | Inhibitor | $K_I$ (nM) | | |
|---|---|---|---|---|
| | | hCA I[a] | hCA II[a] | bCA IV[b] |
| | **B44** | 20 | 4 | 11 |
| | **B45** | 37 | 12 | 25 |
| | **B46** | 22 | 3 | 8 |
| | **B47** | 51 | 9 | 24 |

[a]Human (cloned) isozymes;

[b]From bovine lung microsomes, by the esterase method.

All the new compounds reported here show better CA inhibitory properties as compared to the parent sulfonamide A from which they are obtained, which is a moderately weak inhibitor, similarly to many benzene sulfonamides reported in the literature (Supuran et al., *Exp. Opin. Ther. Patents 12*:217-242, 2002;Supuran et al., *Curr. Med. Chem. Imm., Endoc. Metab. Agents 1*:61-97, 2001; Supuran et al., *Eur. J. Med. Chem.* 30:687-696, 1995; Clare et al., *Eur. J. Med. Chem. 34*:463-474, 1999; Scozzafava et al., *J. Enz. Inhib. 15*:443-453, 2000; Menabuoni et al., *J. Enz. Inhib. 14*: 457-474, 1999).

Thus, the four derivatives of aromatic/heterocyclic amines **B7-B10** (which are the most ineffective CA inhibitors among the new compounds reported here) show affinities of 30- 43 nM against hCA II, of 52-75 nM against bCA IV, and 88-126 nM against the slow isozyme hCA I. Increased inhibitory power is observed for the derivatives obtained from **B** and amino-benzoic acids **11-13** or amino acids 14-**38.** Thus, the most active such compounds are those incorporating β-phenyl-serine **(B20),** hydroxy- (Ser, Thr; **B19-B21)** and mercapto-(Cys, Met; **B22, B23)** amino acids, hydrophobic amino acids (Val, Leu, Ile; **B24-B26),** aromatic amino acids (His, Phe, Tyr, DOPA; **B32-B35);** or dicarboxylic (Asp, Glu, and their corresponding amides, Asn, Gln; **B27-B30)** amino acid moieties. Such compounds possessed inhibition constants in the range of 1-10 nM against hCA II, 7-23 nM against bCA IV and 20-40 nM against hCA I (being more active than the classical inhibitors of type 1-6 mentioned above, which are used as standards in these measurements, Table 2). On the other hand, compound which are less active are the derivatives of aminobenzoic acids **(B11-B13),** those of Gly, Ala, β-Ala and GABA **(B14-B17),** as well as those of Trp, Lys and Arg **(B36-B38),** with inhibition constants in the range of 10-18 nM against hCA II, 23-56 nM against bCA IV and 38-103 nM against hCA I. It is worth noting, however, that these compounds possess the same potency of acetazolamide or methazolamide, clinically used CA inhibitors. Among all the thioureas reported here, the most ineffective CA inhibitor is the proline derivative **B31**, probably due to its relatively bulky nature. Nevertheless, this compound has inhibitor activity comparable to that of the clinically used dichlorophenamide 6. Strong inhibitory properties are, on the other hand, observed for the oligopeptidyl derivatives **B39-B47.** Thus, except for the GlyGly **(B39)** and AspAsp **(B45)** derivatives (which possess the same activity as methazolamide **4** against hCA II), all the other oligopeptidyl thioureas reported herein are very potent inhibitors, with inhibition constants in the range of 1-9 nM against hCA II, 5-25 nM against bCA IV and 12-51 nM against hCA I. Very good inhibitory properties are correlated with the presence of His moieties in such oligopeptidyl thioureas, such as for instance **B40-B42.** It is also interesting to note the influence of the number of aspartyl residues in some of the thioureas prepared in this study, on the CA inhibitory properties of the obtained compounds. Thus, among the Asp, Asp-Asp and $(Asp)_4$ derivatives, the best activity is observed for the simple mono-derivative **(B27),** whereas the most ineffective inhibitor is the dipeptidyl derivative **(B45;** with the $K_I$ against hCA II almost doubled as compared to **B27).** On the other hand, the tetrapeptide derivative **B47** regains much of the hCA II inhibitory activity, but shows a much lower affinity for bCA IV and hCA I. It is difficult to explain at the present moment this intricate behavior. From all these data, the main SAR conclusion is that the best inhibitors are obtained when the molecule of the thiourea is prolonged in the direction of the axis passing through the Zn(II) ion of the enzyme, the sulfonamide sulfur atom as well as the thiourea nitrogen atom of the inhibitor, as was in fact explained theoretically by QSAR studies form our group, for other series of sulfonamide CA inhibitors (Supuran et al., *Eur. J. Med. Chem. 30*:687-696, 1995; Clare et al., *Eur. J. Med. Chem 34*:463-474, 1999). The exceptions to this rule are just the aspartyl derivatives mentioned above, **B27, B45** and **B47.** Among the three CA isozymes herein, the most susceptible to inhibition is hCA II, followed by bCA IV and then hCA I.

EXAMPLE 10

This example illustrates the effect of the new compounds on intraoptic pressure (IOP).

Tonometric measurement of IOP are performed as follows.

Adult male New Zealand albino rabbits weighing 3-3.5 kg are used in the experiments. Three animals are used for each inhibitor studied. The experimental procedures conform to the Association for Research in Vision and

Ophthalmology Resolution on the use of animals. The rabbits are kept in individual cages with food and water provided *ad libitum* and maintained on a 12 h light/dark cycle in a temperature controlled room, at 22-26 °C. Solutions and suspensions of inhibitors (2 %, by weight, as hydrochlorides, or sodium carboxylates) are made in distilled deionized water. The pH of these solutions is in the range of 5.5 - 7.4.

IOP is measured using a Digilab 30R pneumatonometer (BioRad, Cambridge, MA, USA) as described by Maren et al. (Maren et al., *Exp. Eye Res. 55*:73-79, 1992; Brechue et al., *Invest. Ophthalmol. Vis. Sci. 34*: 2581-2587,1993). The pressure readings are matched with two-point standard pressure measurements at least twice each day using a Digilab Calibration verifier. All IOP measurements are done by the same investigator with the same tonometer. One drop of 0.2 % oxybuprocaine hydrochloride (novesine, Sandoz) diluted 1:1 with saline is instilled in each eye immediately before each set of pressure measurements. IOP is measured three times at each time interval, and the means reported. IOP is measured first immediately before drug administration, then at 30 min after the instillation of the pharmacological agent, and then each 30 minutes for a period of 4-6 hours. For all IOP experiments drug is administered to only one eye, leaving the contralateral eye as an untreated control. The ocular hypotensive activity is expressed as the average difference in IOP between the treated and control eye, in this way minimizing the diurnal, seasonal and inter-individual variations commonly observed in the rabbit *(Id.).* All data are expressed as mean ± SE. Ocular hypertension is elicited in the right eye of albino rabbits by the injection of α-chymotrypsin (from Sigma) as described by Sugrue et al. (Sugrue et al., *Br. J. Pharmacol.* 99:59-64, 1990). The IOP of operated animals is checked after approximately four weeks, and animals with an elevated pressure of 30-36 mm Hg are used at least one month after the injection of α-chymotrypsin.

*In vivo* IOP lowering experiments are done in normotensive and glaucomatous rabbits, with many of the new compounds reported here, due to their strong CA II and CA IV inhibitory properties, such as **B7, B10-B13, B15, B18-B22, B25, B27, B28, B33, B37,** and **A40.** Table 3 shows the fall in IOP of normotensive rabbits from starting values of 18.0 ± 2.5 mm Hg, after treatment with one drop (50 µL) of solution containing CA inhibitor at a concentration of 2%. Compounds are in the form of a hydrochloride salt in the case of dorzolamide, **B7** and **B10,** and as sodium salts for the other derivatives. The pH of the solution is shown in the table. Measurements are at 30, 60 and 90 min after administration

Table 3.

| Inhibitor | pH | Δ IOP ± SE[a] (mm Hg) | | |
|---|---|---|---|---|
| | | 30 min | 60 min | 90 min |
| **Dorzolamide** | 5.5 | 2.2±0.15 | 4.1±0.15 | 2.7±0.10 |
| **B7** | 6.5 | 1.2±0.10 | 3.5±0.20 | 0.9±0.25 |
| **B10** | 6.5 | 3.2 ±0.20 | 0.7±0.15 | 0.0 |
| **B11** | 7.0 | 4.2±0.15 | 8.0±0.25 | 4.3±0.10 |
| **812** | 7.0 | 4.0±0.10 | 8.1±0.20 | 9.0±0.30 |
| **B13** | 7.0 | 1.2±0.15 | 7.0±0.15 | 10.3±0.20 |
| **B15** | 7.0 | 3.3±0.10 | 5.2±0.15 | 5.0±0.20 |
| **B18** | 7.5 | 6.4±0.30 | 2.5±0.15 | 2.0±0.10 |
| **B19** | 7.0 | 6.7±0.15 | 9.1±0.25 | 8.3±0.30 |
| **B20** | 7.0 | 8.6±0.20 | 10.3±0.25 | 9.4±0.15 |
| **B22** | 7.0 | 0.0 | 0.0 | 0.0 |
| **B25** | 7.0 | 5.4±0.15 | 6.0±0.20 | 6.8±0.30 |
| **B27** | 7.0 | 1.7 ±0.10 | 2.5 ± 0.20 | 2.3 ± 0.20 |
| **B28** | 7.0 | 6.3 ±0.15 | 9.1 ± 0.30 | 7.4±0.20 |
| **B33** | 7.0 | 4.3±0.15 | 4.0±0.30 | 2.9±0.25 |
| **B37** | 7.0 | 0.0 | 0.0 | 0.0 |
| **B40** | 7.0 | 1.5 ±0.15 | 3.0±0.30 | 4.6±0.20 |

[a]$\Delta IOP = IOP_{control\ eye} - IOP_{treated\ eye}$ (N = 3).

The following may be noted regarding the data of Table 3: (i) some of the new derivatives, such as **B19, B20** and **B28,** provoke a very strong and rapid decrease of IOP when applied topically, with IOP lowering of 6.3 - 8.6 mm Hg at 30 min after administration (compared with 2.2 mm Hg IOP lowering after dorzolamide), which at 1 hour amounts to 9.1 - 10.3 mm Hg (versus 4.1 mm Hg of dorzolamide), and this potent effect is maintained at 1.5 hours (7.4 -9.4 mm

Hg); furthermore, IOP returns to baseline values after 5-6 hours from administration (see Fig. 2). Thus, such new inhibitors are much more potent hypotensor agents, and their effect is much longer, than that of the standard drug dorzolamide. It is interesting to note that two of these very effective IOP lowering agents mentioned above are the Ser and β-phenylserine derivatives, whereas the third one is the Asn derivative. More surprisingly, the very similar Asp derivative (**B27**) is a modest IOP hypotensor agent. This demonstrates that very small structural modifications (e.g., replacement of the $CONH_2$ moiety with COOH) leads to a completely different pharmacological profile for the two derivatives containing them, although their CA inhibitory properties are rather similar; (ii) compounds such as **B11, B12, B15, B18, B25** and **B33** show an IOP lowering of 3.3 - 6.4 mm Hg at 30 min after administration (being more effective than dorzolamide), whereas at one hour their effect is in the range of 2.5 - 8.1 mm Hg, and at 90 min of 2.9 - 9.0 mm Hg. Thus, although more effective than the standard drug at 30 min after administration, some of these agents show a diminished effect at later times, probably due to their washing away from the ciliary processes. This may be due to an improper balance between lipo- and hydrophilicity for some of these compounds; (iii) derivatives such as **B7, B10, B22, B27, B37** and **B40** show a modest effect in the decrease of IOP after administration, with IOP lowering of 0.0 - 3.2 mm Hg after 30 min, 0.0 - 3.5 mm Hg after 60 min, and 0.0 - 4.6 mm Hg after 90 min; (iv) compounds such as **B13** show a modest reduction of IOP after 30 min, but show continued effectiveness at times of one hour and longer after administration (Table 3 and Figure 2).

In considering duration of action, it is important to correlate the physico-chemical properties of the new CA inhibitors reported here (Table 1), and their *in vivo* activity as topical hypotensor agents. It is most interesting that some of the newly obtained compounds, although acting as very potent hCA II (and bCA IV) inhibitors, show an unexpectedly small IOP lowering effect via the topical route, whereas structurally very similar derivatives act as strong hypotensors. Compare, for example, **B19** and **B22,** which differ minimally, by the presence of an OH moiety in the first and an SH in the second compound; or, as in the example mentioned above, the Asp and Asn derivatives, **B27** and **B28.** Maren (Maren, *J. Glaucoma 4*:49-62, 1995) has noted previously that, at a minimum, for a sulfonamide to act as an effective IOP lowering agent, the compound should possess a modest (but not insignificant) lipid solubility (attributable to its unionized form), accompanied by a good water solubility (eventually conferred by the presence of ionizable groups of appropriate $pK_a$). As seen from data of Tables 1 and 2, the more effective topical hypotensors, such as **B13, B19, B20** or **B28,** possessed just this type of balanced physico-chemical properties, which lead to good accession rates across the cornea, followed by sustained inhibition of the ciliary processes enzyme. In contrast, the Cys **(B22)** or Asp **(B27)** derivatives, although effective CA inhibitors and possessing a very good water solubility, these compounds have a low hydrophobicity (due to the presence of the second ionizable moiety, SH in the first case, and COOH in the second one) and, as a result, a decreased penetration through the cornea (see, for instance the low $k_{in}$ values shown in Table 1). The same may be true other derivatives herein, such as for instance the Lys or oligopeptidyl derivatives **(B37** or **B40),** which again show relatively modest IOP lowering, although they are extremely efficient in vitro CA inhibitors. One must also mention that a very hydrophobic inhibitor (such as ethoxzolamide 6) is also ineffective topically, although it possesses very high accession rates through the cornea *(Id.).* Still, this property also allows its rapid diffusibility into red blood cells, and, as a result, its washing away from the ciliary processes due to the blood circulation *(Id).* This might explain why some of the compounds reported here (such as the α-Ph-Gly derivative **B18)** initially show a potent IOP lowering (at 30 min after administration), whereas this effect rapidly diminishes at later periods after the administration. One may observe that **B18** is the most hydrophobic among the inhibitors investigated in some detail in this study (Table 2), and this probably leads not only to very good accession rates across the cornea, but also to its rapid clearing from the ciliary processes, similarly to ethoxzolamide 6 mentioned above. Thus, this series of CA inhibitors proves in a clear-cut manner that in order to obtain hypotensors with prolonged duration of action via the topical route, the physico-chemical and enzyme inhibitory properties must be fine-tuned in a very precise manner, and that such compounds should possess a right balance between lipo- and hydrophobicity, in addition to acting as potent hCA II inhibitors and possessing an acceptable water solubility.

Example 11

This example illustrates the distribution of CA inhibitors in ocular fluids and tissues.

The determination of drug distribution in ocular fluids and tissues is performed as follows. The general procedure of Maren et al. is followed (Maren et al., *Exp. Eye Res.* I55:73-79*,* 1992; Brechue et al., *Invest. Ophthalmol. Vis. Sci.* 34:2581-2587, 1993). The animals are killed with an intracardiac injection. Aqueous humor (both posterior and anterior chamber fluids) are withdrawn. Then, the cornea and anterior uvea (iris plus attached ciliary body) are dissected, rinsed well with water, blotted, weighed and put into 1-2 mL of water. For isolation of the ciliary processes, intact anterior uvea rings are placed on a parafilm covered piece of polystyrene foam in a Petri dish. The tissue is wetted with normal saline and dissected under a microscope, when ciliary processes are liberated from their attachment to the iris, cut, weighed and put in 0.5 mL of distilled water. The tissue from 4 eyes (average weight of 8 mg/ eye) is pooled for drug analysis. Samples are boiled for 5 minutes (in order to denature CA, and free drug from the E-I complex),

diluted and then incubated with a known amount of enzyme. The activity of the free enzyme and in the presence of the inhibitor are determined as described above. A calibration curve is used in order to determine the fractional inhibition in the different tissues, as described earlier *(Id.)*.

Table 4 shows the drug distribution in ocular fluids and tissues after topical administration of compounds **B13** and **B20.** Ocular tissue concentrations (μM) are assessed after one and two hours following corneal application of one drop (50 μL) of 2% solution of inhibitors **B13** and **B20** in albino rabbits. Dorzolamide hydrochloride 1 served as the reference compound.

Table 4

| Time (h) | Drug concentration (μM)* | | |
|---|---|---|---|
| | Cornea | Aqueous humor | Ciliary process |
| 1(.HCl) | | | |
| 1h<br>2h | 105 ± 5<br>39 ± 4 | 32 ± 3<br>21 ± 2 | 15 ± 3<br>6 ± 1 |
| **B13** | | | |
| 1h<br>2h | 123 ± 9<br>76 ± 5 | 204 ± 13<br>62 ± 4 | 50 ± 7<br>23 ± 2 |
| **B20** | | | |
| 1h<br>2h | 133 ± 10<br>70 ± 9 | 205 ± 12<br>53 ± 5 | 49 ± 5<br>15 ± 1 |

* Mean ± standard deviation (n= 3).

It is seen from Table 4 that at one and two hours after topical administration of the drug, high levels of inhibitors are found in the cornea, aqueous humor and ciliary processes. Based on the inhibition constant of these compounds, the fractional inhibition estimated in these tissues and fluids is of 99.5-99.9 % (Supuran et al., *Curr. Med. Chem.- Imm., Endoc. Metab. Agents, 1*: 61-97, 2001), which indicates that the IOP decrease is indeed due to CA inhibition. Furthermore, as also seen from the data of Table 4, the new compounds reported here, tend to concentrate in the aqueous humor (concentrations of around 204 -205 μM are detected after one hour from administration), whereas dorzolamide reaches much lower concentrations (for example, 32 μM after one hour). High concentrations of the inhibitor are maintained at 2 hours from administration too. Thus, the strong and long lasting IOP lowering properties of the new compounds reported here are probably due to this concentrating effect reached mainly in the aqueous humor, but which is also present in the cornea and ciliary processes.

Thus, we report here a novel class of sulfonamides, obtained by reaction of 4-isothiocyanatobenzenesulfonamide with amines, amino acids or oligopeptides. Many of the newly reported derivatives show very good water solubility, at nearly neutral pH values, whereas their lipid solubility, hydrophobicity (LogP) as well as accession rates across the cornea are appropriate for eliciting efficient topical IOP lowering activity. Some of these new CA inhibitors possessed affinities in the nanomolar range for isozymes hCA II and bCA IV, acting as effective enzyme inhibitors *in vitro.* Several of the most active inhibitors strongly lower IOP pressure in normotensive and glaucomatous rabbits, showing a prolonged duration of action as compared to dorzolamide. The new compounds reported here might lead to the development of more efficient antiglaucoma drugs from the class of the sulfonamide CA inhibitors.

All references cited in this specification are hereby incorporated by reference. The discussion of the references herein is intended merely to summarize the assertions made by their authors and no admission is made that any of the references or statements therein constitute prior art relevant to patentability. Applicants reserve the right to challenge the accuracy and pertinency of the cited references.

The description of the invention is merely exemplary in nature and, thus, variations that do not depart from the gist of the invention are intended to be within the scope of the invention. Such variations as would occur to one of skill in the art in light of the description are not to be regarded as a departure from the spirit and scope of the invention, which is defined in the appended claims.

## Claims

**1.** A thiourea compound or a salt thereof for use as a medicament for reduction of intraocular pressure wherein the

thiourea compound has structure:

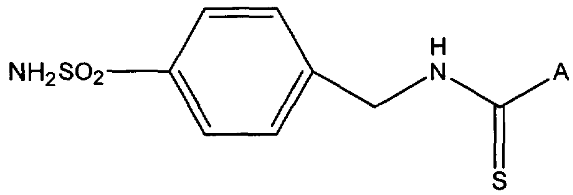

where A is selected from the group consisting of:

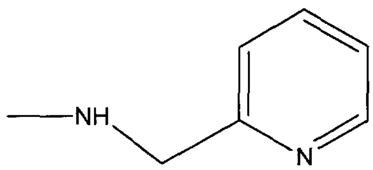

,

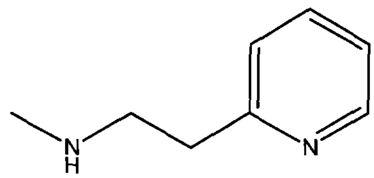

,

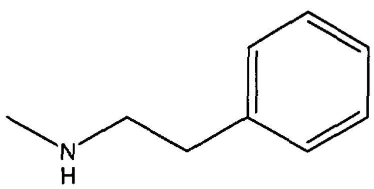

,

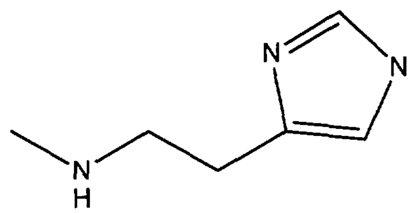

,

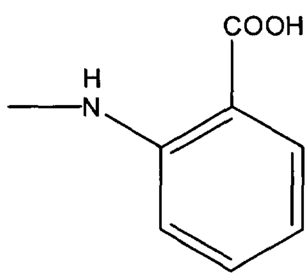

,

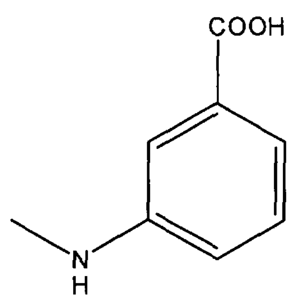

,

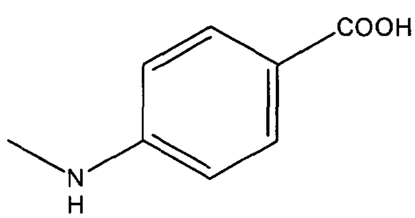

,

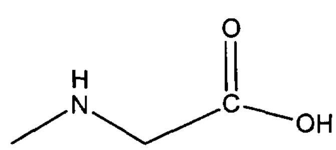

,

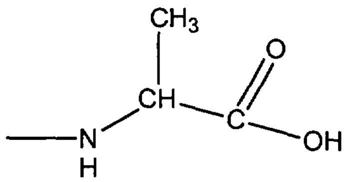,

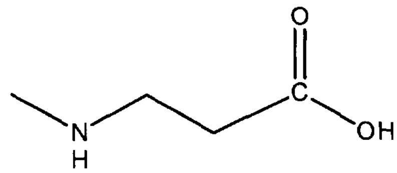,

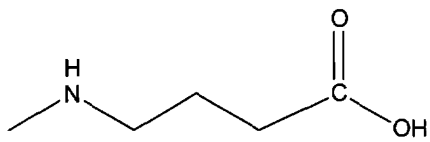,

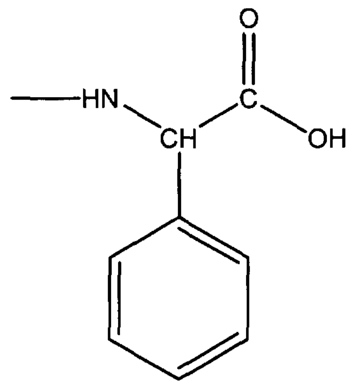,

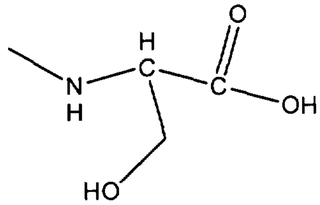,

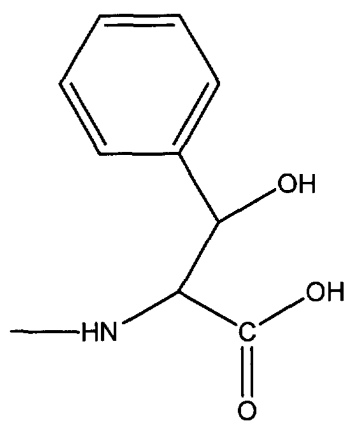,

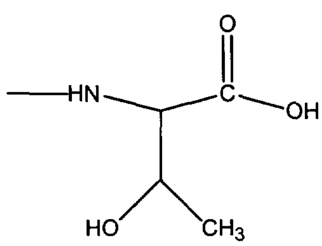,

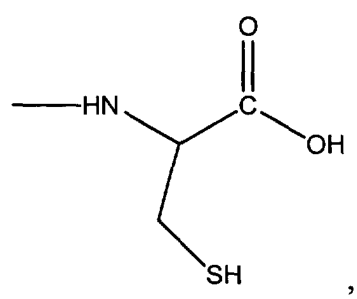,

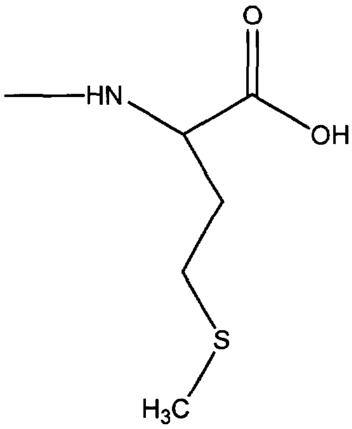
,
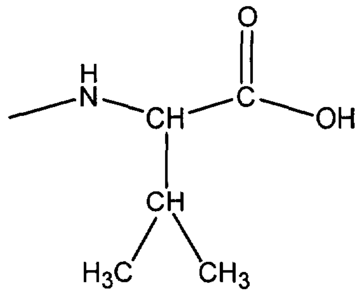
,
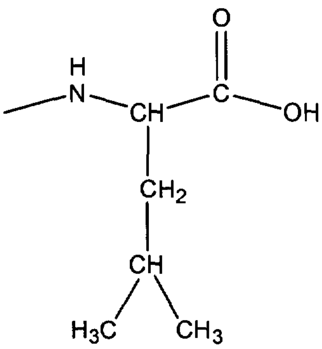
,
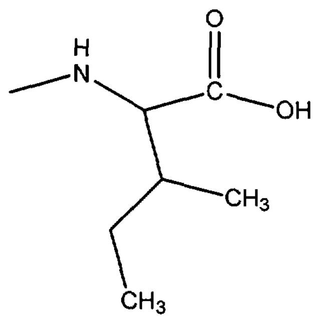
,
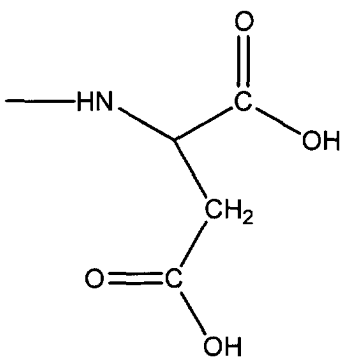
,
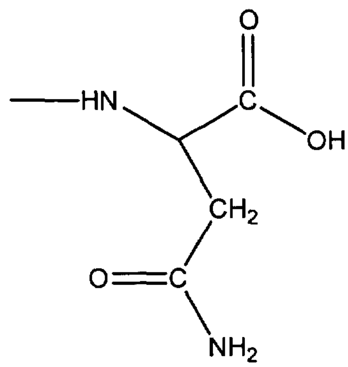
,

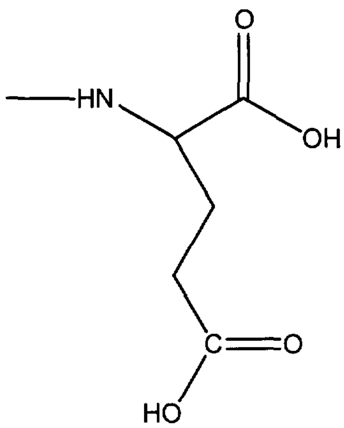

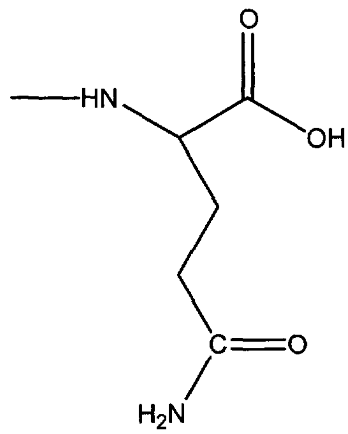

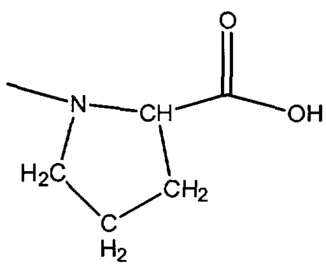

,

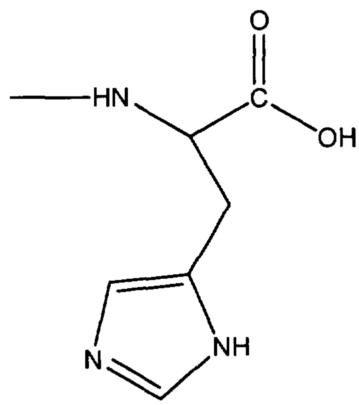

,

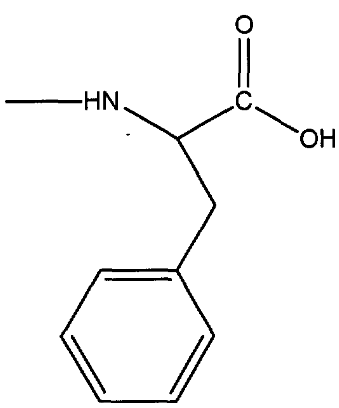

,

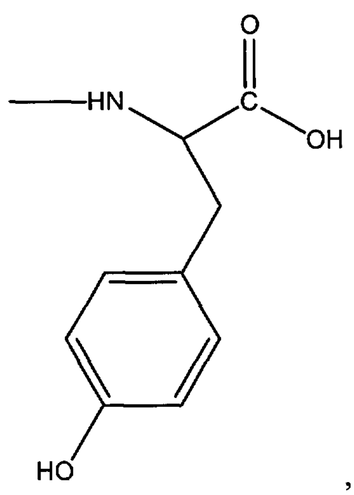

,

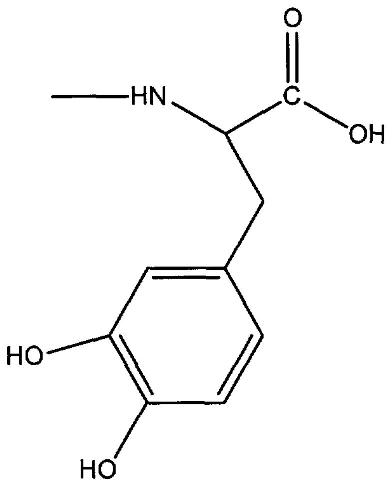
,

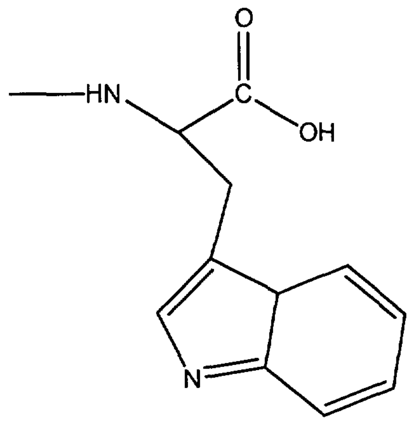
,

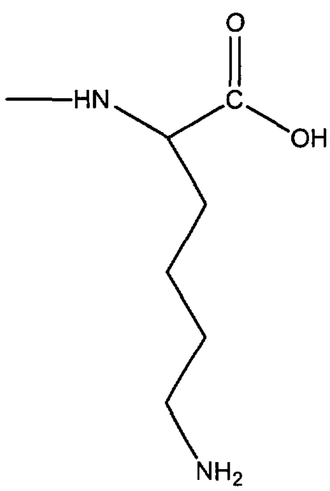
,

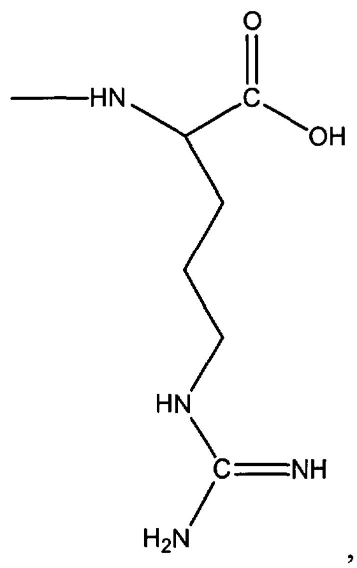
,

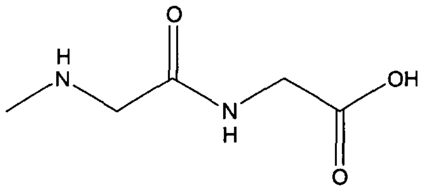
,

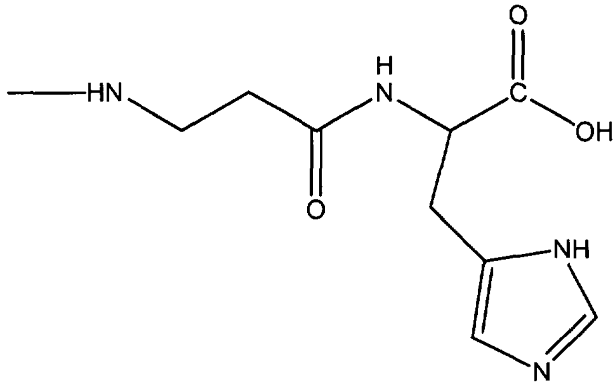
,

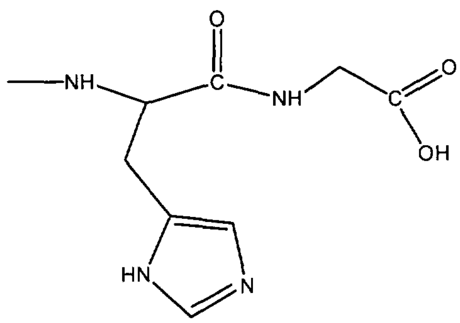
,

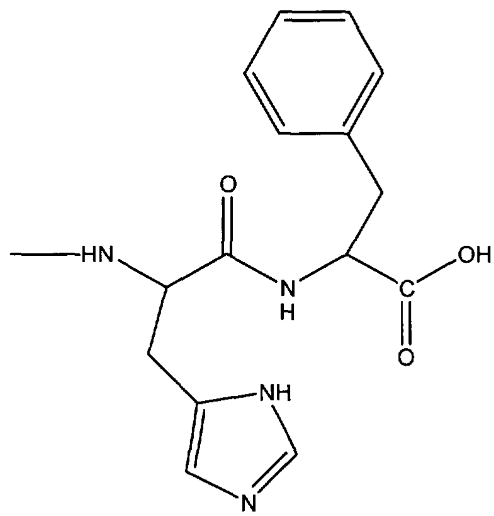
,

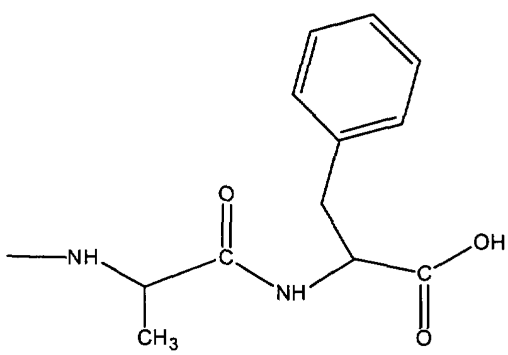
,

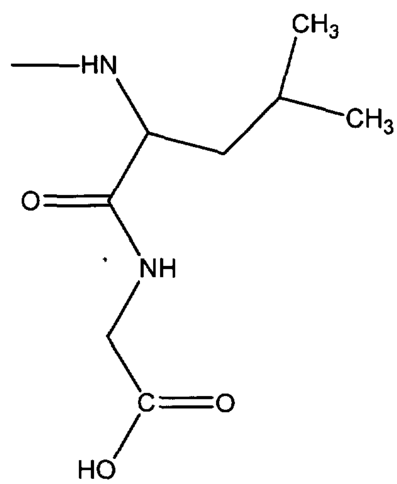
,

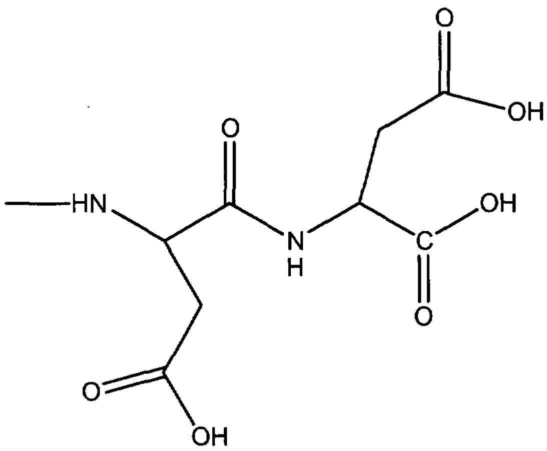

,

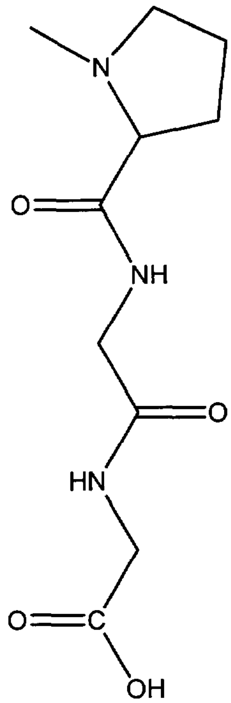

,

and

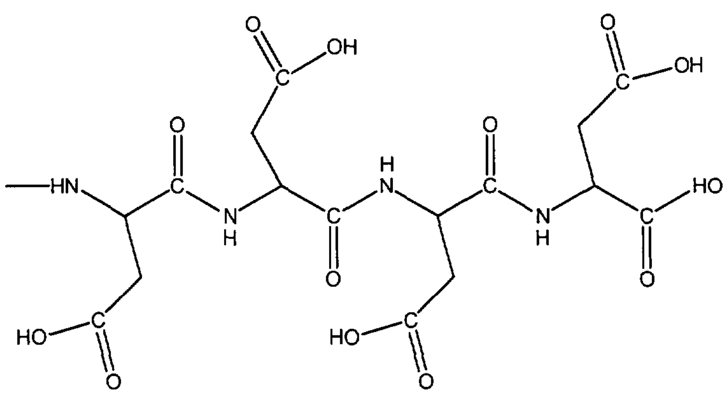

.

2. A thiourea compound or salt thereof according to claim 1 wherein reduction of intraocular pressure comprises treating glaucoma or ocular hypertension.

3. A thiourea compound or salt thereof according to claim 1 wherein said thiourea compound or salt thereof is in an aqueous solution having a pH of from about 5.5 to about 7.4.

4. A thiourea compound or salt thereof according to claim 1 wherein said salt compound is a hydrochloride salt or a sodium carboxylate salt.

5. A thiourea compound or salt thereof according to claim 4 wherein the thiourea compound is in an aqueous solution at a concentration of 2% weight per total weight of solution.

6. Use of a thiourea compound or a salt thereof in the manufacture of a medicament for the reduction in intraocular

pressure wherein the thiourea compound has structure:

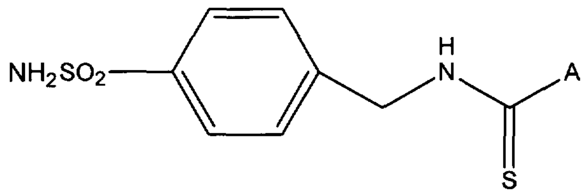

where A is selected from the group consisting of:

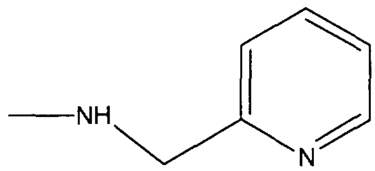
,

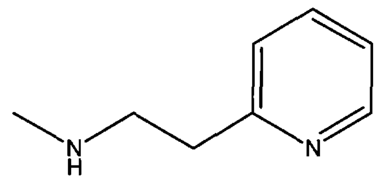
,

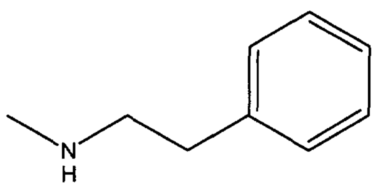
,

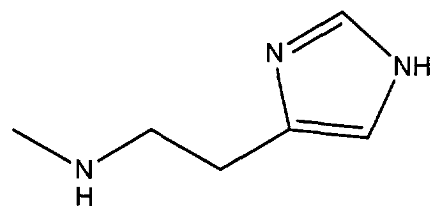
,

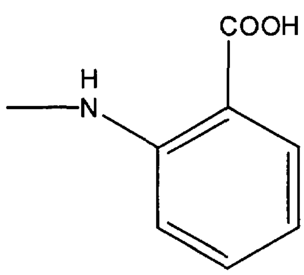
,

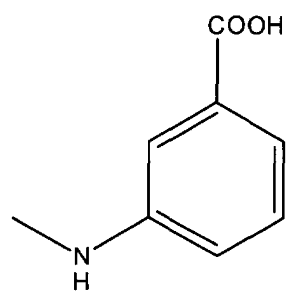
,

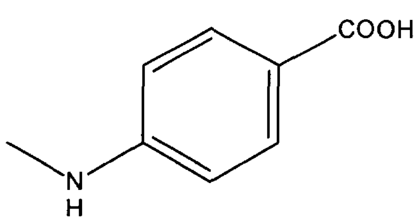
,

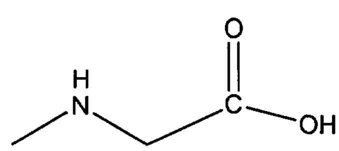
,

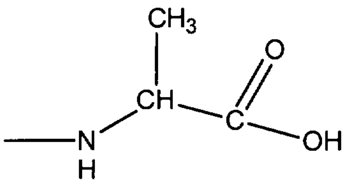

,

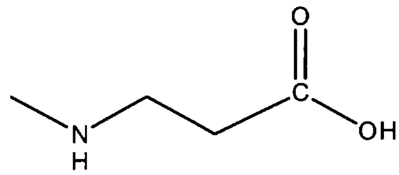

,

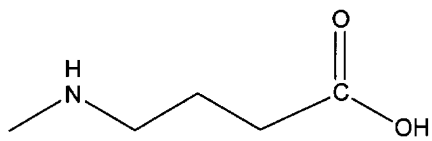

,

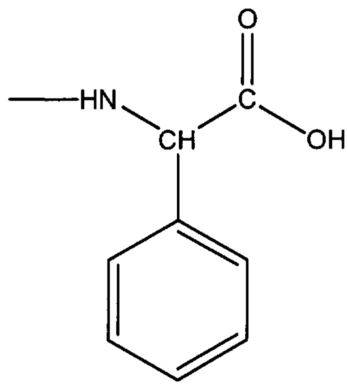

,

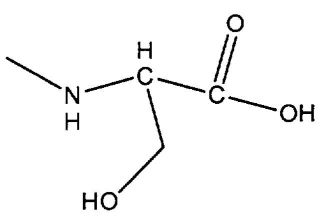

,

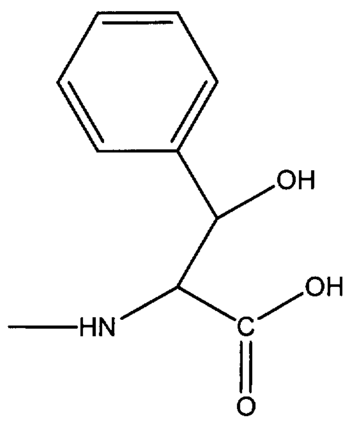

,

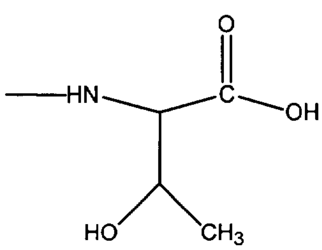

,

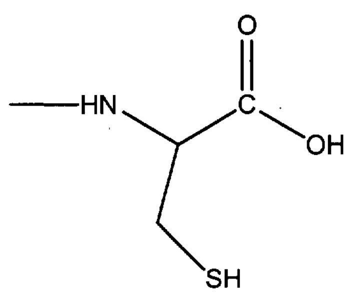

,

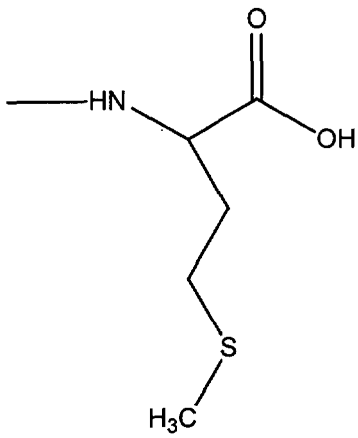

,

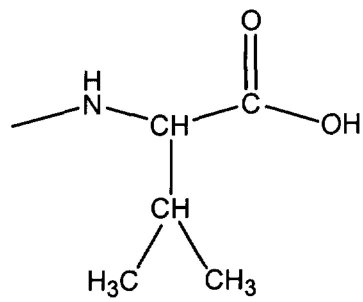

,

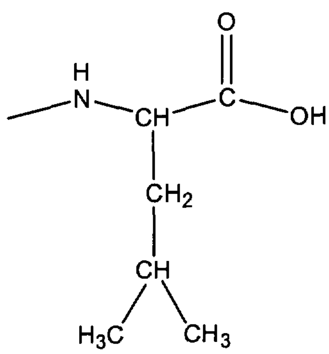

,

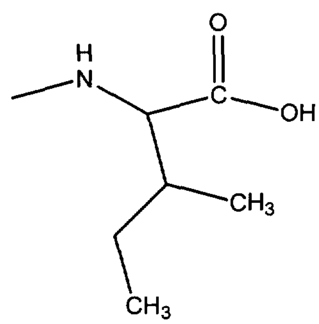

,

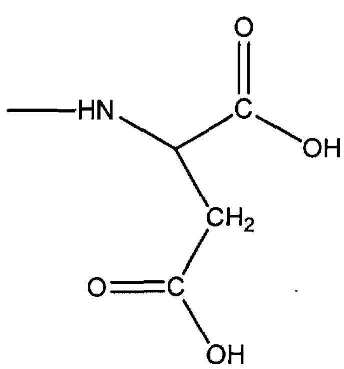

,

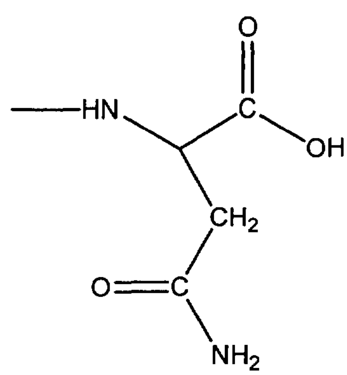

,

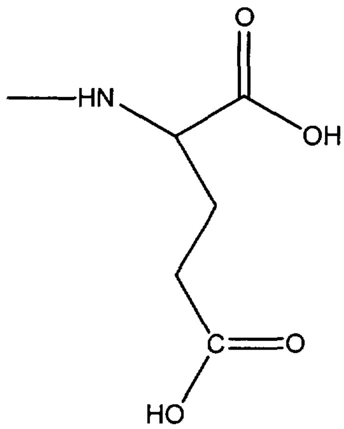

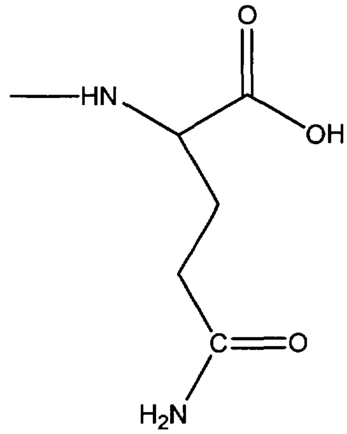

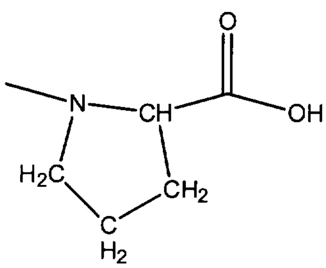

,

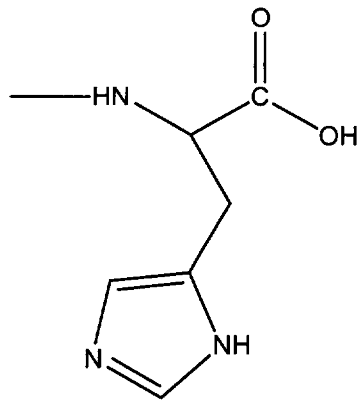

,

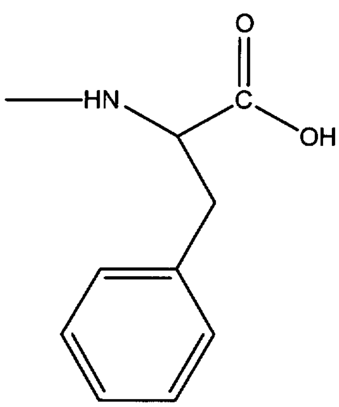

,

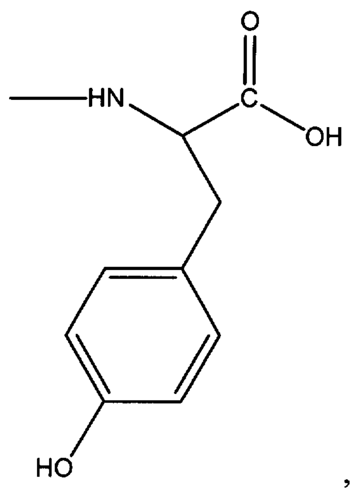

,

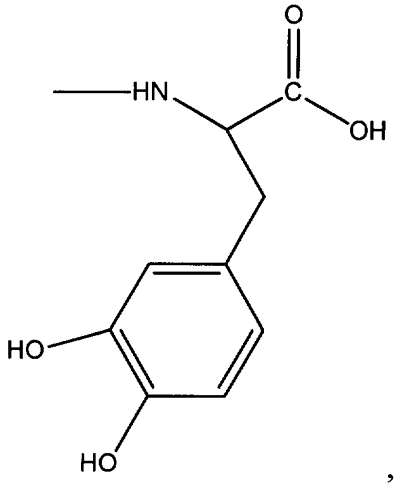

,

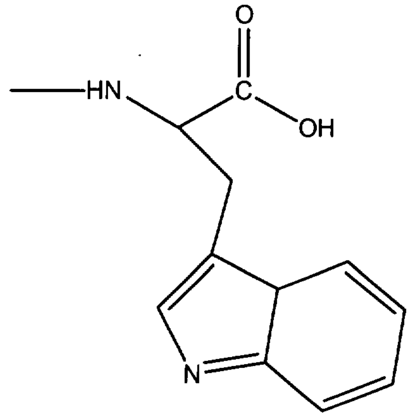

,

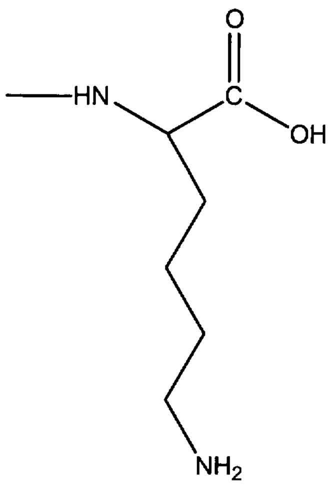

,

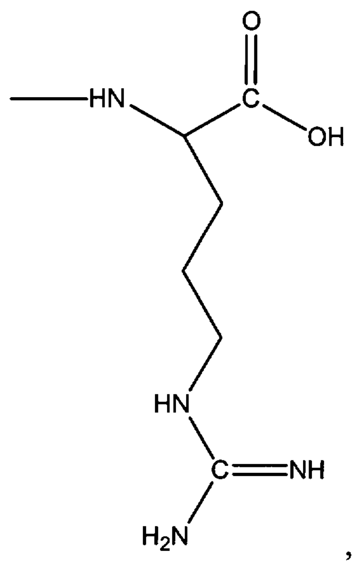

,

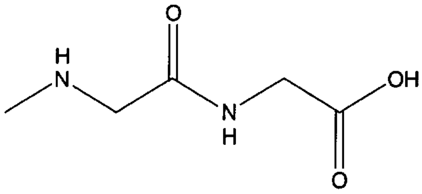

,

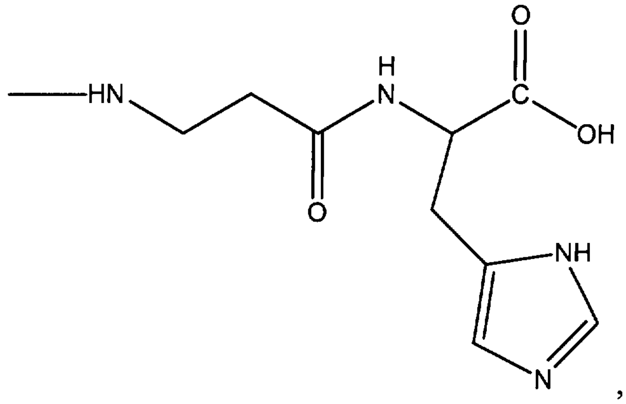

,

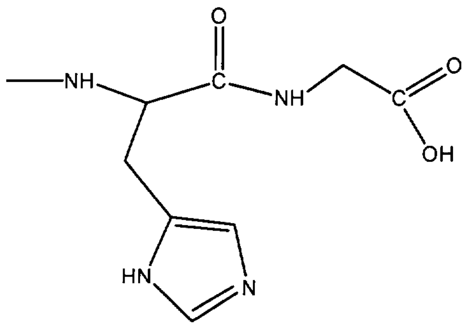
,

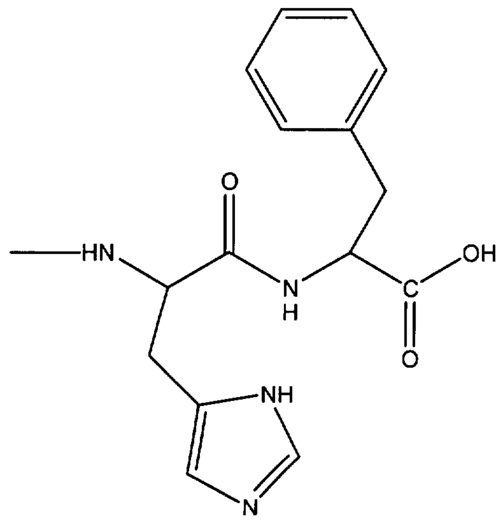
,

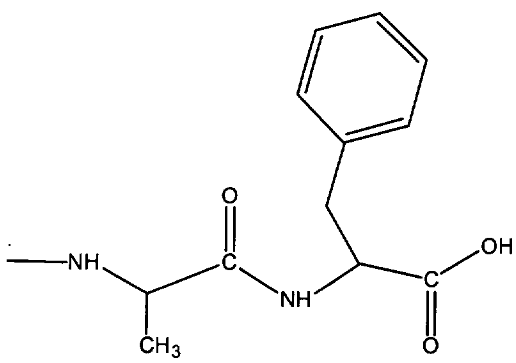
,

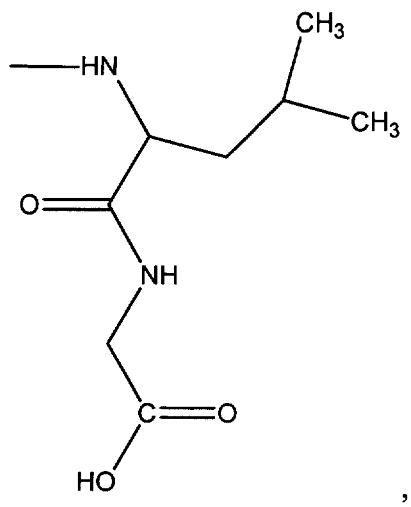
,

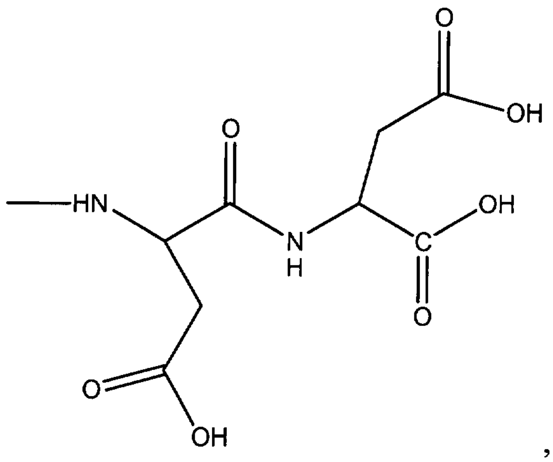

,

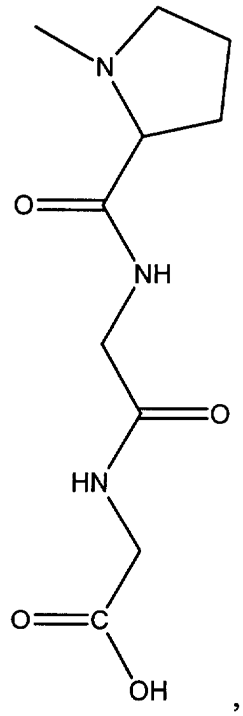

,

and

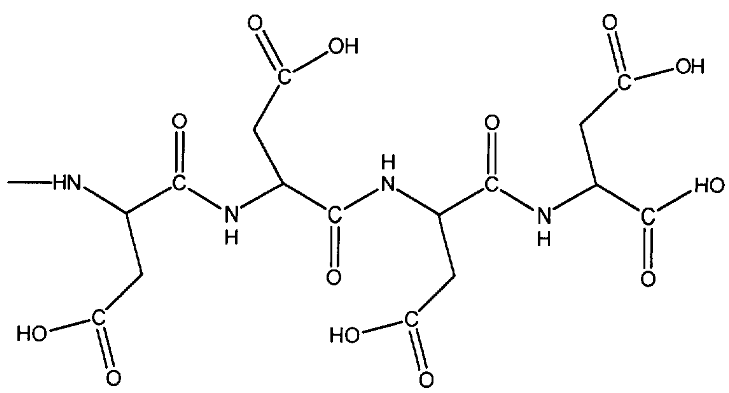

.

**7.** Use of a thiourea compound or salt thereof according to claim 6 wherein reduction of intraocular pressure comprises treating glaucoma or ocular hypertension.

**8.** Use of a thiourea compound or salt thereof according to claim 6 wherein said thiourea compound or salt thereof is in an aqueous solution having a pH of from about 5.5 to about 7.4.

**9.** Use of a thiourea compound or salt thereof according to claim 6 wherein said salt is a hydrochloride salt or a sodium carboxylate salt.

**10.** Use of a thiourea compound or a salt thereof according to claim 6 wherein the thiourea compound or salt thereof is in an aqueous solution at a concentration of 2% weight per total weight of solution.

**11.** An eye drop composition comprising a carbonic anhydrase inhibitory compound or a salt thereof in an aqueous formulation suitable for topical application for reduction of intraocular pressure, wherein the carbonic anhydrase inhibitory compound has structure:

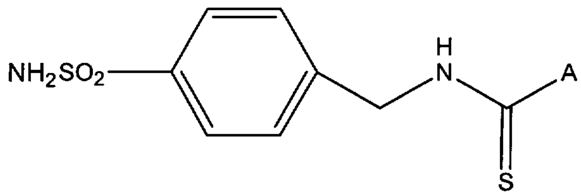

where A is selected from the group consisting of:

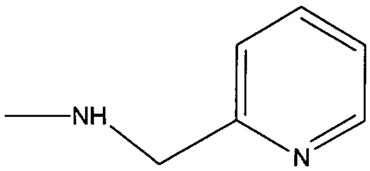

,

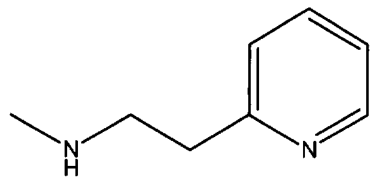

,

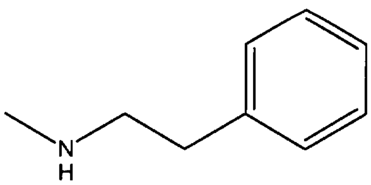

,

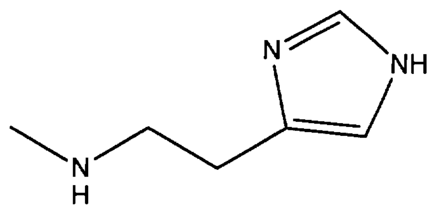

,

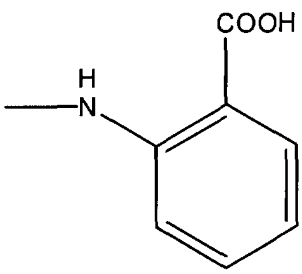

,

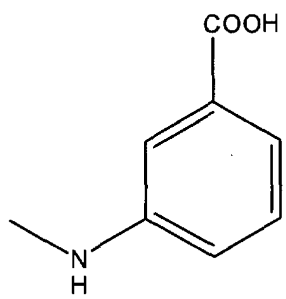

,

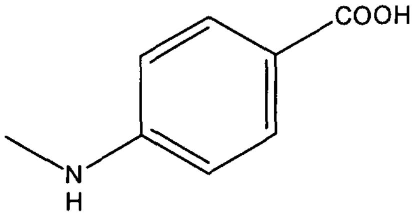

,

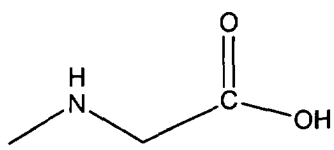

,

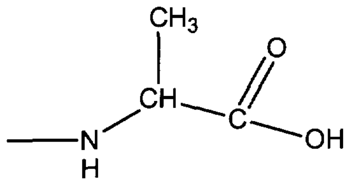 ,

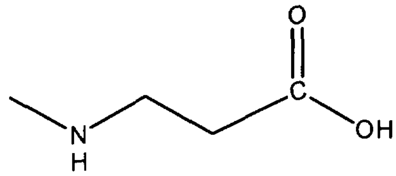 ,

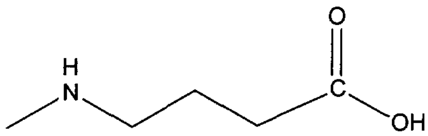 ,

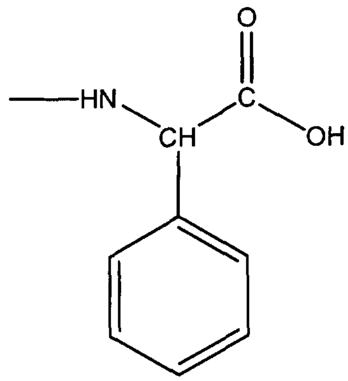 ,

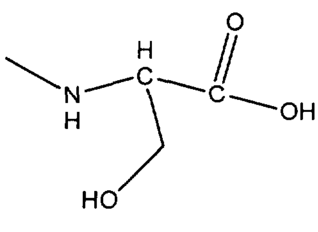 ,

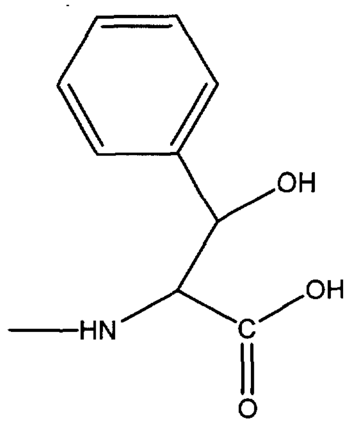 ,

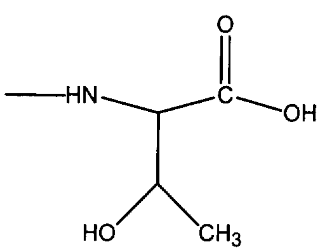 ,

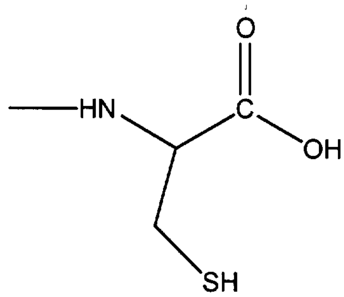 ,

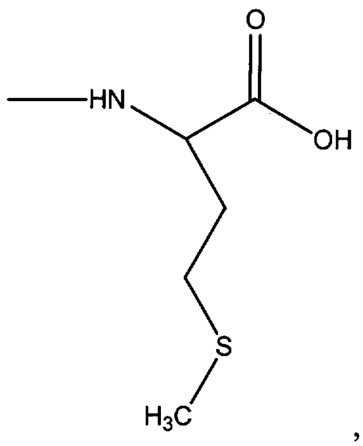,

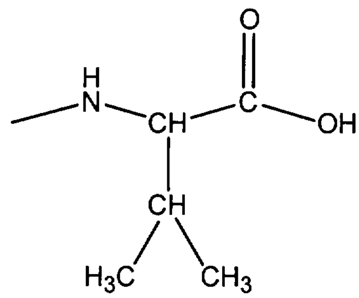,

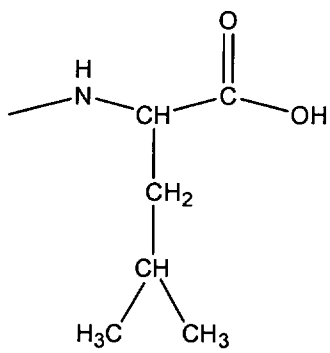,

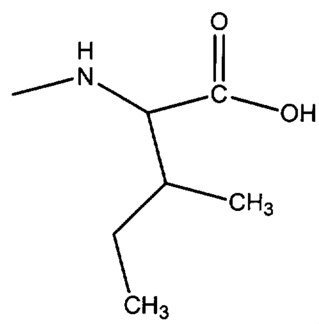,

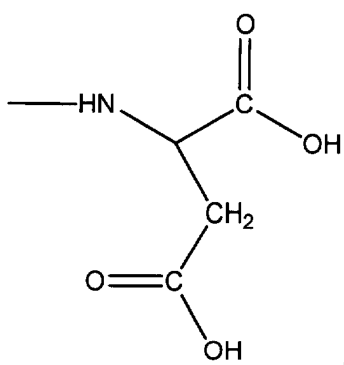,

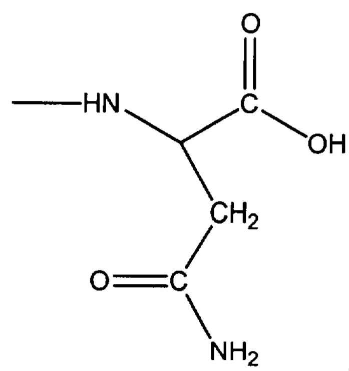,

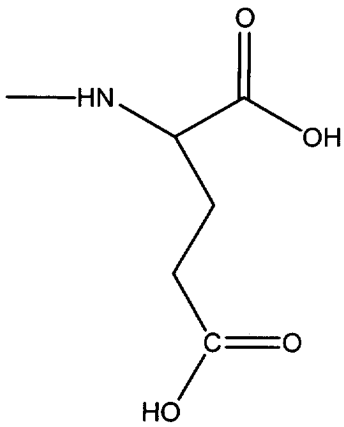

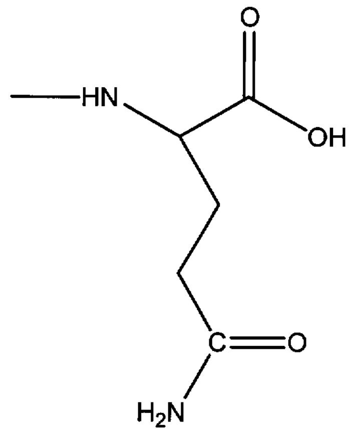

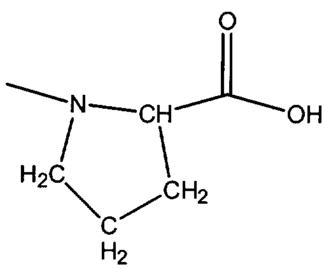
,

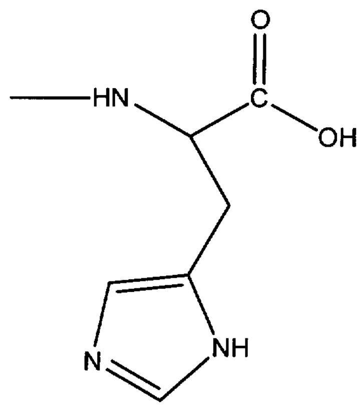
,

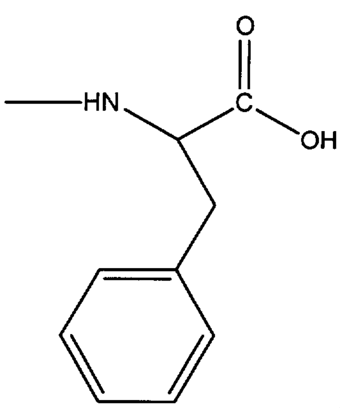
,

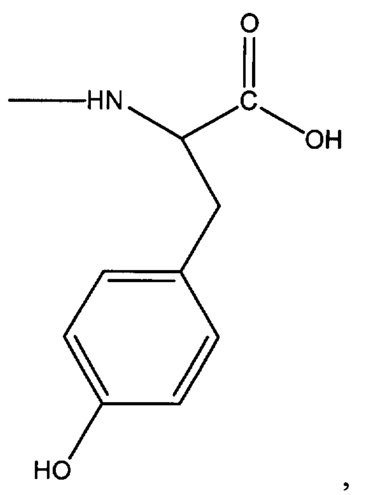
,

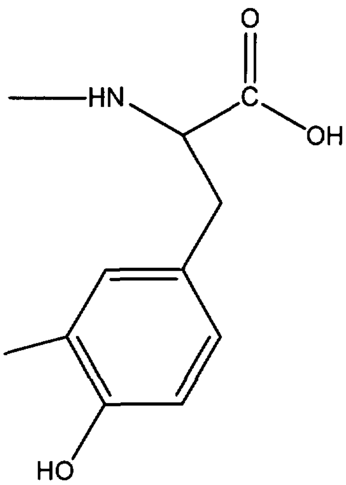

,

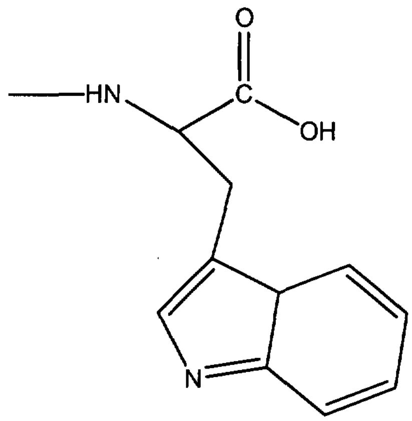

,

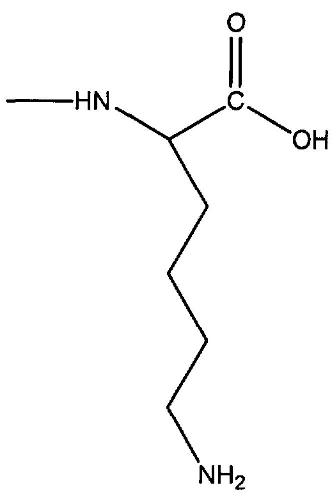

,

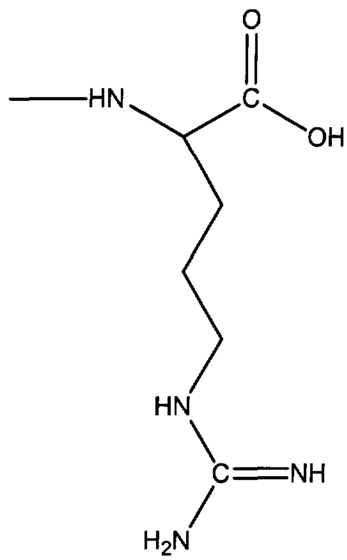

,

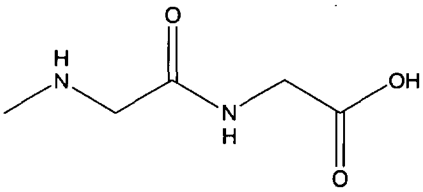

,

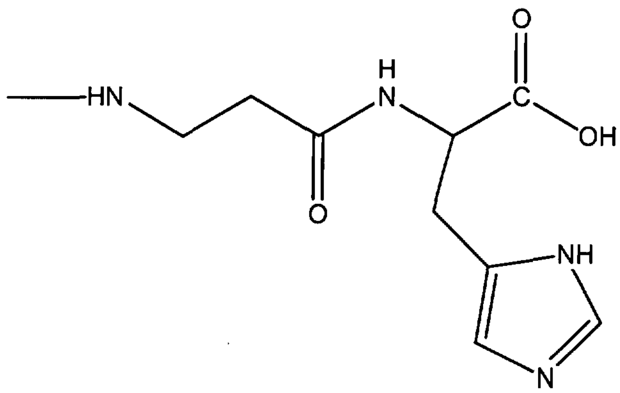

,

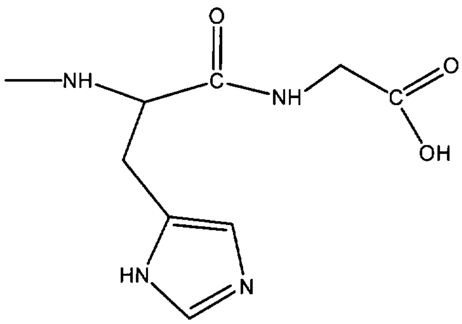

,

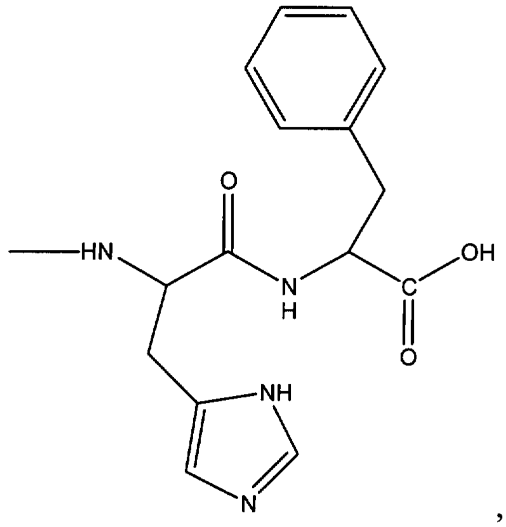

,

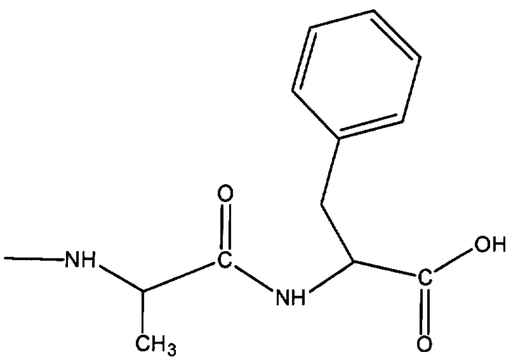

,

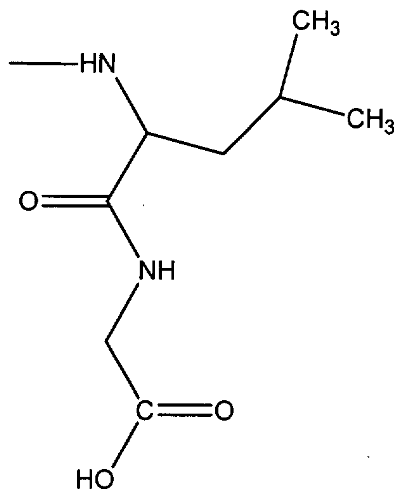

,

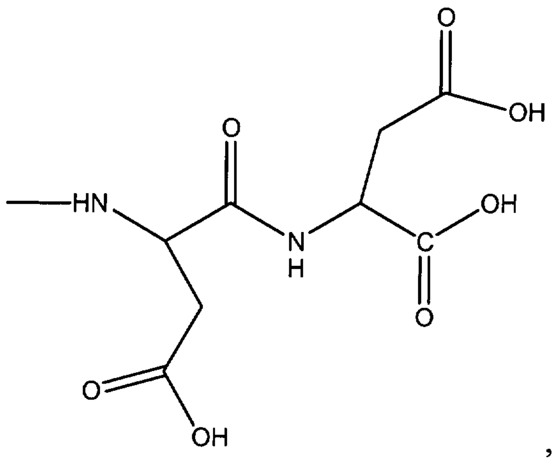

,

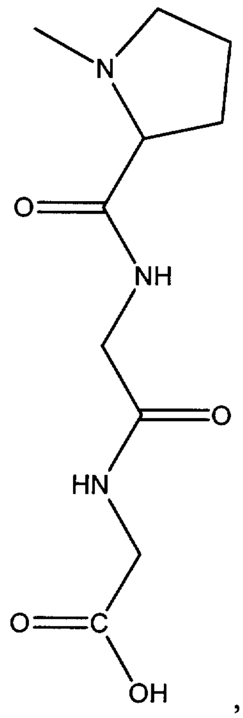

,

and

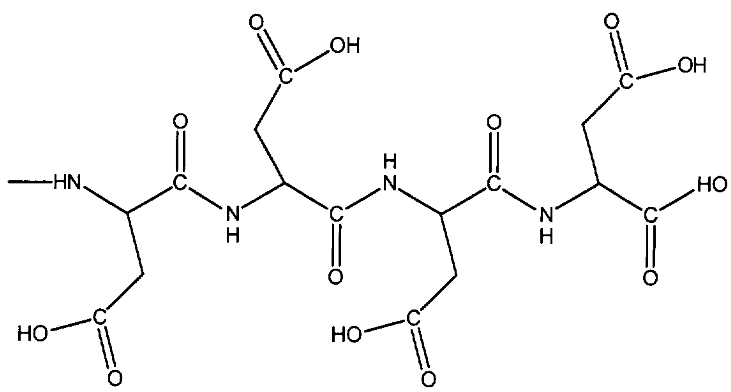

.

**12.** An eye drop composition according to claim 11 wherein reduction of intraocular pressure comprises treating glaucoma or ocular hypertension.

**13.** An eye drop composition according to claim 11 wherein said carbonic anhydrase inhibitory compound or salt thereof is in an aqueous solution having a pH of from about 5.5 to about 7.4.

**14.** An eye drop composition according to claim 13 wherein said salt is a hydrochloride salt or a sodium carboxylate salt.

**15.** An eye drop composition according to claim 11 wherein the carbonic anhydrase inhibitory compound or salt thereof is in an aqueous solution at a concentration of 2% weight per total weight of solution.

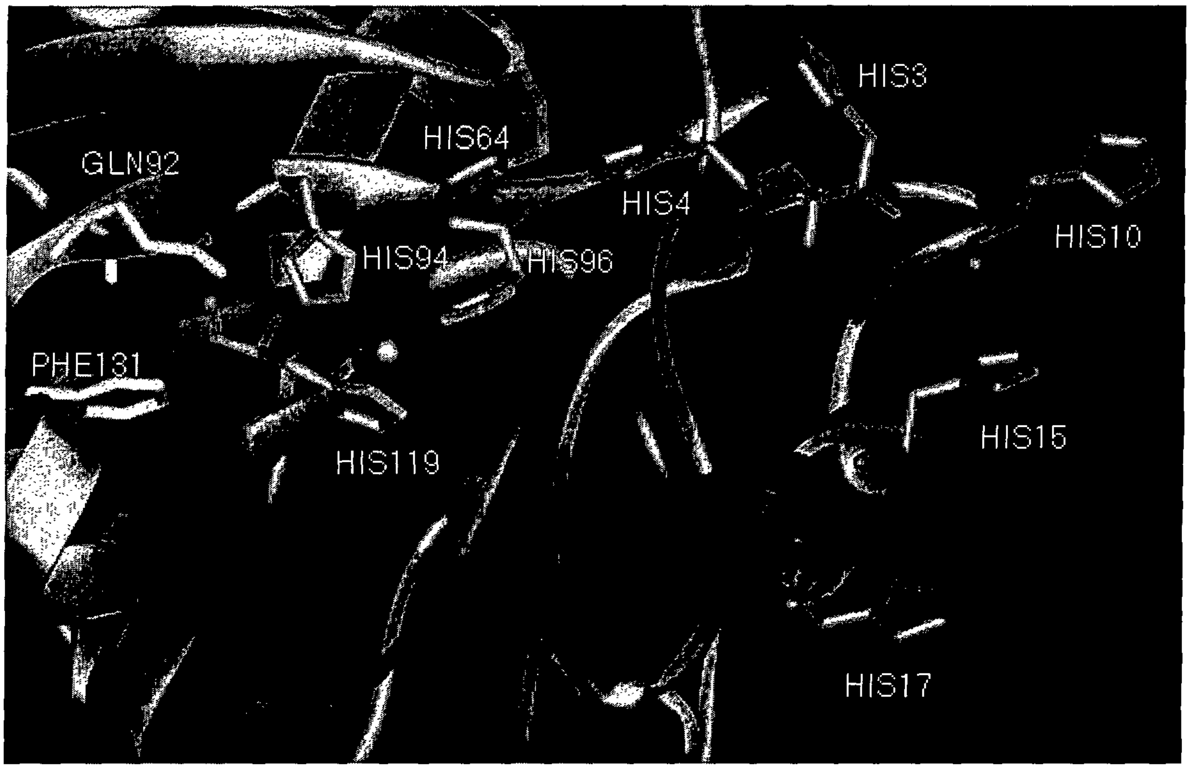
HIS3
HIS64
GLN92
HIS4
HIS10
HIS94
HIS96
PHE131
HIS15
HIS119
HIS17

FIGURE 1.

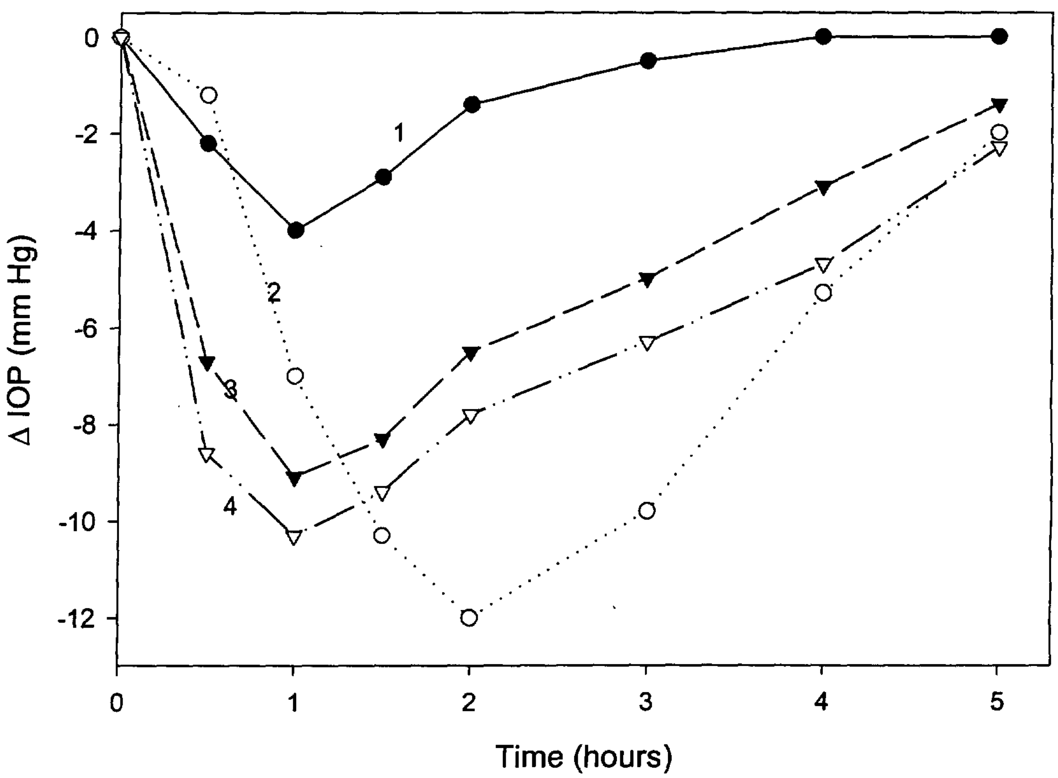


FIGURE 2.

European Patent Office

# EUROPEAN SEARCH REPORT

Application Number
EP 02 42 5687

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | MARTIN-D, RIECHE-A, IYER-R-N: "Synthetische Senfölbildner, IV" ARCHIV DER PHARMAZIE, vol. 296, no. 10, 1963, pages 641-650, XP008015179 * page 641 - page 642 * * page 645 - page 648 * --- | 1,2 | A61K31/17 A61K31/18 A61K31/03 A61K31/415 A61K31/401 A61K31/405 A61K31/192 A61K31/44 A61K38/05 A61K31/197 A61K31/198 A61K38/07 A61K38/06 A61K31/4172 A61P27/06 |
| Y,D | CASINI A ET AL: "CARBONIC ANHYDRASE INHIBITORS: WATER-SOLUBLE 4-SULFAMOYLPHENYLTHIOUREAS AS TOPICAL INTRAOCULAR PRESSURE-LOWERINGAGENTS LONG-LASTING EFFECTS" JOURNAL OF MEDICINAL CHEMISTRY, AMERICAN CHEMICAL SOCIETY, US, vol. 43, 2000, pages 4884-4892, XP001024011 ISSN: 0022-2623 * page 4884 - page 4887; figure SCHEME1 * --- | 1-15 | |
| Y | SUPURAN C T ET AL: "Carbonic anhydrase inhibitors - Part 49: Synthesis of substituted ureido and thioureido derivatives of aromatic/heterocyclic sulfonamides with increased affinities for isozyme I" EUROPEAN JOURNAL OF MEDICINAL CHEMISTRY, EDITIONS SCIENTIFIQUE ELSEVIER, PARIS, FR, vol. 33, no. 2, 1 February 1998 (1998-02-01), pages 83-93, XP004122409 ISSN: 0223-5234 * page 89 * * page 91 * ----- | 1-15 | TECHNICAL FIELDS SEARCHED (Int.Cl.7) A61K A61P |

The present search report has been drawn up for all claims

| Place of search | Date of completion of the search | Examiner |
|---|---|---|
| MUNICH | 1 April 2003 | Schifferer, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)